(19) Europäisches Patentamt / European Patent Office / Office européen des brevets

(11) **EP 3 488 773 B1**

(12) **EUROPEAN PATENT SPECIFICATION**

(45) Date of publication and mention of the grant of the patent:
**11.08.2021 Bulletin 2021/32**

(21) Application number: **16909557.7**

(22) Date of filing: **22.07.2016**

(51) Int Cl.:
*A61B 5/02* (2006.01)  *A61B 5/024* (2006.01)
*A61B 5/00* (2006.01)  *G09B 19/00* (2006.01)
*G06Q 50/10* (2012.01)  *G16H 50/30* (2018.01)

(86) International application number:
**PCT/JP2016/071646**

(87) International publication number:
**WO 2018/016082 (25.01.2018 Gazette 2018/04)**

(54) **INFORMATION PROCESSING DEVICE, DIGESTION RATE ESTIMATION METHOD, INFORMATION PROCESSING SYSTEM, AND DIGESTION RATE ESTIMATION PROGRAM**

INFORMATIONSVERARBEITUNGSVORRICHTUNG, VERFAHREN ZUR SCHÄTZUNG DER VERDAUUNGSRATE, INFORMATIONSVERARBEITUNGSSYSTEM UND PROGRAMM ZUR SCHÄTZUNG DER VERDAUUNGSRATE

DISPOSITIF DE TRAITEMENT D'INFORMATIONS, PROCÉDÉ D'ESTIMATION DE TAUX DE DIGESTION, SYSTÈME DE TRAITEMENT D'INFORMATIONS, ET PROGRAMME D'ESTIMATION DE TAUX DE DIGESTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.05.2019 Bulletin 2019/22**

(73) Proprietor: **Fujitsu Limited**
**Kawasaki-shi, Kanagawa 211-8588 (JP)**

(72) Inventors:
• **OYA, Takuro**
**Kawasaki-shi**
**Kanagawa 211-8588 (JP)**
• **YAGINUMA, Yoshinori**
**Kawasaki-shi**
**Kanagawa 211-8588 (JP)**

(74) Representative: **Haseltine Lake Kempner LLP**
**Cheapside House**
**138 Cheapside**
**London**
**EC2V 6BJ (GB)**

(56) References cited:
**EP-A1- 2 992 816**  **EP-A1- 3 354 193**
**WO-A1-96/03076**  **WO-A1-96/03076**
**WO-A1-2008/061788**  **WO-A1-2016/092707**
**JP-A- 2003 173 375**  **JP-A- 2007 048 180**
**JP-A- 2012 045 191**  **JP-A- 2013 220 110**

• **KIMIYO YAMAGUCHI: "Sesshoku ga Myakuhaku-to ni Oyobosu Eikyo -Kenkosha 12-mei ni Tsuite", Journal of universities' nursing research, vol. 1, no. 2, 1978, pages 14-18, XP055370968,**

**Description**

Technical Field

The present invention relates to an information processor, a digestibility estimation method, an information processing system, and a digestibility estimation program.

Background Art

In recent years, service providers have been conducting a service to sense a situation or behavior of a user and deliver information corresponding to the situation or behavior to the user.

For example, there is disclosed a service to provide useful information to a user according to a digestibility of consumed food (NPL 1). This service uses a technology to estimate a digestibility (hungry level) from an acetone concentration by measuring the acetone concentration from the breath. The user may know, through a portable information terminal, optimal timing for meal or exercise based on the estimated hungry level.

Citation List

Patent Literature

PTL 1: Japanese National Publication of International Patent Application No. 10-504739
PTL 2: Japanese Laid-open Patent Publication No. 2004-138
PTL 3: Japanese Laid-open Patent Publication No. 2009-201805
PTL 4: Japanese Laid-open Patent Publication No. 2011-115508
PTL 5: Japanese Laid-open Patent Publication No. 2008-61790

Non Patent Literature

NPL 1: "Diet Support Service by DOCOMO - Display Calories from Meal Photo and Measure Hungry Level from Breath", [online], ITmedia Inc., [searched on July 6, 2015], Internet <URL: http://www.itmedia.co.jp/mobile/articles/1110/06/news128.html>

Reference may be made to EP 3 354 193 A1, which relates to an information processing device including a first receiver configured to receive, from a heart rate signal measuring device carried by a user, a plurality of heart rate signal information items measured at predetermined time intervals, a second receiver configured to receive information of meal start time and meal end time of the user, a characteristic amount calculator configured to identify digestion end time indicating a time when a digestion of the food ends, based on the heart rate of the user at the meal start time and a trend indicated by the plurality of heart rate signal information items and indicating that the heart rate of the user increases and decreases over time after an end of the meal, and a digestion rate estimator configured to estimate a digestion rate based on the meal end time and the identified digestion end time. WO 2016/092707 A1 discloses meal intake estimation based on heart rate data.

Summary of Invention

Technical Problem

The device for measuring the acetone concentration described above is not small enough to be carried all the time. Also, acetone remaining in the device has to be removed upon every measurement. Therefore, it is difficult to provide a service corresponding to the digestibility of consumed food to the user in real time by monitoring the acetone concentration.

In one aspect of the present invention, it is an object of the present invention to provide an information processor, a digestibility estimation method, an information processing system, and a digestibility estimation program, which are capable of providing a service corresponding to a digestibility of consumed food to a user in real time.

Solution to Problem

The present invention is defined by the independent claims, to which reference should now be made. Specific embodiments are defined in the dependent claims. There is disclosed herein an information processor for estimating a digestibility of food consumed by a user, including: a first receiver for receiving a plurality of pieces of heart rate information measured at specified time intervals from a heart rate signal measurement device carried by the user; a second receiver for receiving information of a meal start time and a meal finish time of the user; a line calculation unit for calculating, based on a trend indicated by the plurality of pieces of heart rate information, in which the heart rate rises with time after meal and then drops, a first-order approximation line that approximates the trend; a correction unit for correcting the first-order approximation line based on a trend indicated by the plurality of pieces of heart rate information in which the heart rate rises with time during meal and then drops; a time specification unit for specifying a digestion finish time indicating the time when digestion of the food is completed, based on the corrected first-order approximation line and the heart rate at the meal start time; and a digestibility estimation unit for estimating the digestibility at the current time based on the meal finish time and the specified digestion finish time.

Advantageous Effects of Invention

According to one embodiment of the present invention, an information processor, a digestibility estimation method, an information processing system, and a digestibility estimation program that are capable of providing a service corresponding to a digestibility of consumed food to a user in real time may be provided.
The invention is defined by an apparatus according to independent claim 1, a method according to independent claim 7 and a program according to independent claim 9.

Brief Description of Drawings

[FIG. 1] FIG. 1 is a diagram illustrating an example of an information processing system 100 in Example 1.
[FIG. 2] FIG. 2 is a diagram illustrating an example of a hardware configuration of an information processor in Example 1.
[FIG. 3] FIG. 3 is a graph illustrating an example of a temporal change in heart rate between before meal and after meal.
[FIG. 4] FIG. 4 is a flowchart (Part 1) illustrating an example of a digestibility estimation method executed by the information processor 10.
[FIG. 5] FIG. 5 is a diagram illustrating an example of an initial state of a heart rate management table 12a.
[FIG. 6] FIG. 6 is a diagram illustrating an example where information is stored in the heart rate management table 12a.
[FIG. 7] FIG. 7 is a diagram illustrating an example of an initial state of a meal time management table 12b.
[FIG. 8] FIG. 8 is a diagram illustrating an example where information is stored in the meal time management table 12b in S102.
[FIG. 9] FIG. 9 is a diagram illustrating an example of an initial state of a feature amount management table 12c.
[FIG. 10] FIG. 10 is a diagram illustrating an example where information is stored in the feature amount management table 12c in S103.
[FIG. 11] FIG. 11 is a diagram illustrating an example where information is stored in the feature amount management table 12c in S104.
[FIG. 12] FIG. 12 is a diagram for explaining a method for estimating a digestibility with a first-order approximation line.
[FIG. 13] FIG. 13 is a diagram illustrating an example where information is stored in the feature amount management table 12c in S105.
[FIG. 14] FIG. 14 is a diagram (Part 1) for explaining the background of processing in S107.
[FIG. 15] FIG. 15 is a diagram (Part 2) for explaining the background of the processing in S107.
[FIG. 16] FIG. 16 is a flowchart illustrating a specific example of the processing in S107 in Example 1.
[FIG. 17] FIG. 17 is a diagram (Part 1) for explaining a method for calculating an area of a figure extracted from a waveform of a first wave.
[FIG. 18] FIG. 18 is a diagram (Part 2) for explaining the method for calculating the area of the figure extracted from the waveform of the first wave.
[FIG. 19] FIG. 19 is a diagram (Part 3) for explaining the method for calculating the area of the figure extracted from the waveform of the first wave.
[FIG. 20] FIG. 20 is a diagram illustrating an example of an initial state of a first-order approximation line management table 12d.
[FIG. 21] FIG. 21 is a diagram illustrating an example where information is stored in the first-order approximation line management table 12d in S201.
[FIG. 22] FIG. 22 is a diagram illustrating an example of the corrected value management table.
[FIG. 23] FIG. 23 is a diagram illustrating an example where information is stored in the first-order approximation line management table 12d in S202.

[FIG. 24] FIG. 24 is a diagram illustrating an example where information is stored in the first-order approximation line management table 12d in S203.

[FIG. 25] FIG. 25 is a diagram illustrating an example where information is stored in the feature amount management table 12c in S108.

[FIG. 26] FIG. 26 is a diagram illustrating an example where information is stored in the feature amount management table 12c in S109.

[FIG. 27] FIG. 27 is a flowchart (Part 2) illustrating an example of the digestibility estimation method executed by the information processor.

[FIG. 28] FIG. 28 is a diagram illustrating an example of an initial state of a digestibility estimation management table 12f.

[FIG. 29] FIG. 29 is a diagram illustrating an example where information is stored in the digestibility estimation management table 12f in S111.

[FIG. 30] FIG. 30 is a diagram illustrating an example where information is stored more than once in the digestibility estimation management table 12f.

[FIG. 31] FIG. 31 is a flowchart illustrating an example of a method for generating a corrected value management table.

[FIG. 32] FIG. 32 is a diagram illustrating a calculation example of a corrected value α.

[FIG. 33] FIG. 33 is a diagram illustrating an example of an information processing system 200 in Example 2.

[FIG. 34] FIG. 34 is a diagram for explaining a method for correcting a first-order approximation line in Example 2.

[FIG. 35] FIG. 35 is a flowchart illustrating a specific example of processing in S107 in Example 2.

[FIG. 36] FIG. 36 is a diagram illustrating an example of a corrected value management table in Example 2.

[FIG. 37] FIG. 37 is a diagram illustrating an example of an initial state of a first-order approximation line management table 12g.

[FIG. 38] FIG. 38 is a diagram illustrating an example where information is stored in the first-order approximation line management table 12g in S202a.

[FIG. 39] FIG. 39 is a diagram illustrating an example where information is stored in the first-order approximation line management table 12g in S203a.

[FIG. 40] FIG. 40 is a diagram illustrating an example of an information processing system 300 in Example 3.

[FIG. 41] FIG. 41 is a flowchart (Part 1) illustrating an example of a digestibility estimation method executed by the information processor 10c in Example 3.

[FIG. 42] FIG. 42 is a flowchart (Part 2) illustrating an example of the digestibility estimation method executed by the information processor 10c in Example 3.

[FIG. 43] FIG. 43 is a diagram illustrating an example of an information processing system 400 in Example 4.

[FIG. 44] FIG. 44 is a diagram illustrating an example of a method for displaying information on a recommended restaurant.

Description of Embodiments

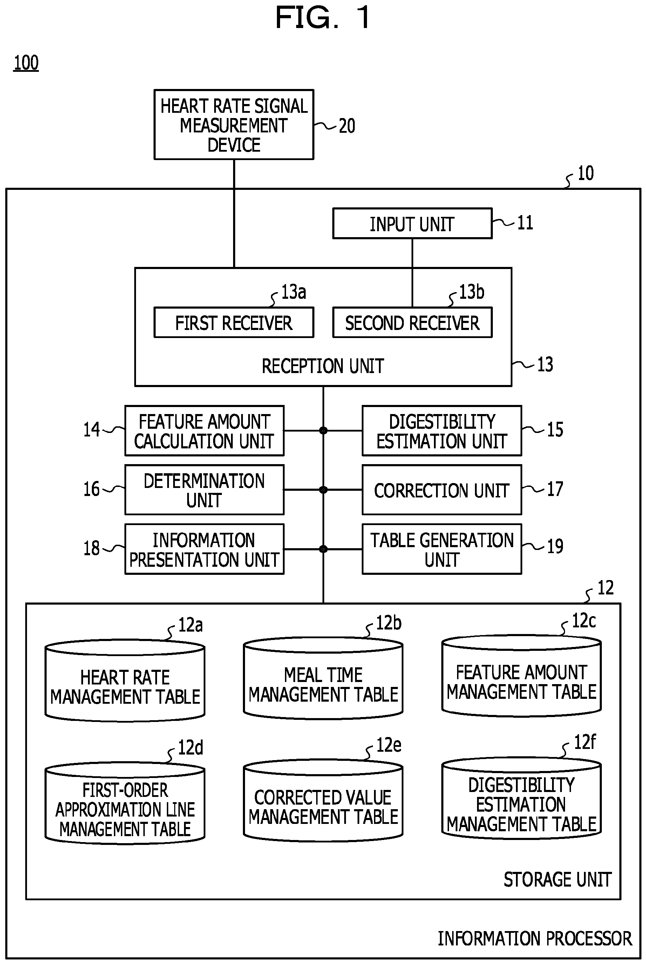

With reference to FIGs. 1 to 42, an embodiment of the present invention is specifically described below.

(EXAMPLE 1)

FIG. 1 is a diagram illustrating an example of an information processing system 100 in Example 1. As illustrated in FIG. 1, the information processing system 100 includes an information processor 10 and a heart rate signal measurement device 20.

The information processor 10 is a computer carried by a user who receives services according to a digestibility of food consumed in the stomach. The information processor 10 is a device that estimates a digestibility from a heart (pulse) rate received from the heart rate signal measurement device 20, and provides services to the user based on the estimated result. The information processor 10 is, for example, a computer such as a smartphone, a cell-phone, a tablet terminal, a laptop PC (Personal Computer), and a wearable computer. The wearable computer is a computer that may be worn and carried around by the user, which is, for example, a wristwatch-type device.

The heart rate signal measurement device 20 is a device for measuring a heart (pulse) rate, which is, for example, a wristwatch-type heart rate meter. The heart rate signal measurement device 20 may measure a heart rate of the user wearing the heart rate signal measurement device 20 at specified time intervals. The heart rate signal measurement device 20 may transmit information on the measured heart rate to the information processor 10.

The information processing system 100 may also be realized as a wearable computer including the information processor 10 and the heart rate signal measurement device 20. The information processing system 100 may be easily worn at all times by being realized as the wearable computer.

Hereinafter, description is given of functions of respective units included in the information processor 10.

As illustrated in FIG. 1, the information processor 10 includes an input unit 11, a storage unit 12, a reception unit 13, a feature amount calculation unit 14, a digestibility estimation unit 15, a determination unit 16, a correction unit 17, an information presentation unit 18, and a table generation unit 19.

The input unit 11 has a function to receive input of various information from the user.

The storage unit 12 is hardware that stores data and programs used for processing to be executed by the information processor 10. For example, the storage unit 12 may store a heart rate management table 12a, a meal time management table 12b, a feature amount management table 12c, a first-order approximation line management table 12d, a corrected value management table 12e, a digestibility estimation management table 12f, and the like to be executed by the information processor 10. The respective tables are described in detail later.

The storage unit 12 is, for example, an HDD (Hard Disk Drive), an SSD (Solid State Drive), a RAM (Random Access Memory), a flash memory, or a ROM (Read Only Memory), or may be a combination thereof. For example, the HDD, the SSD, the RAM, and an NAND-type flash memory may be used to store data. On the other hand, an NOR-type flash memory and the ROM may be used to store programs (digestibility estimation program and the like). The storage unit 12 may also be configured using a plurality of storage devices depending on the intended use, required memory capacity, or the like.

The reception unit 13 includes a first receiver 13a and a second receiver 13b. The first receiver 13a receives information on a plurality of heart rate signals measured at specified time intervals from the heart rate signal measurement device 20. Such heart rate signal information is information associating the heart rate of the user with the time of measurement of the heart rate. The second receiver 13b receives information on a meal start time and a meal finish time from the user through the input unit 11. The meal start time is the time when the user starts his/her meal. The meal finish time is the time when the user finishes his/her meal.

The feature amount calculation unit 14 calculates various parameters used to calculate a digestibility, based on the plurality of heart rate signal information received from the heart rate signal measurement device 20 and the information on the meal start time and the meal finish time of the user. Such various parameters are hereinafter collectively referred to as the "feature amount". Further details about various feature amounts and a calculation method thereof are described later. Furthermore, the feature amount calculation unit 14 also has a function as a line calculation unit using the calculated feature amount to calculate a first-order approximation line, as well as a function as a time specification unit using the first-order approximation line to specify a digestion finish time. Further details about the first-order approximation line and a method for specifying the digestion finish time are also described later.

The digestibility estimation unit 15 estimates a digestibility based on the various feature amounts calculated by the feature amount calculation unit 14. A method for estimating a digestibility is described later.

The determination unit 16 executes various kinds of determination processing among processing to be executed by the information processor 10.

The correction unit 17 executes processing of correcting the slope of the first-order approximation line with the corrected value management table 12e. This correction processing is described in detail later.

The information presentation unit 18 presents information on the digestibility to the user when the determination unit 16 determines that the information is to be presented to the user. To be more specific, the information presentation unit 18 causes a display device 66 to be described later to display characters or images regarding the information to be presented, for example. Alternatively, the information presentation unit 18 transmits an instruction signal to instruct information presentation to a device carried by the user.

The table generation unit 19 executes processing of generating the corrected value management table 12e used to correct the slope of the first-order approximation line that is one of the plurality of feature amounts. Further details about the corrected value management table 12e and a generation method thereof are described later.

Next, a hardware configuration of the information processor 10 is described.

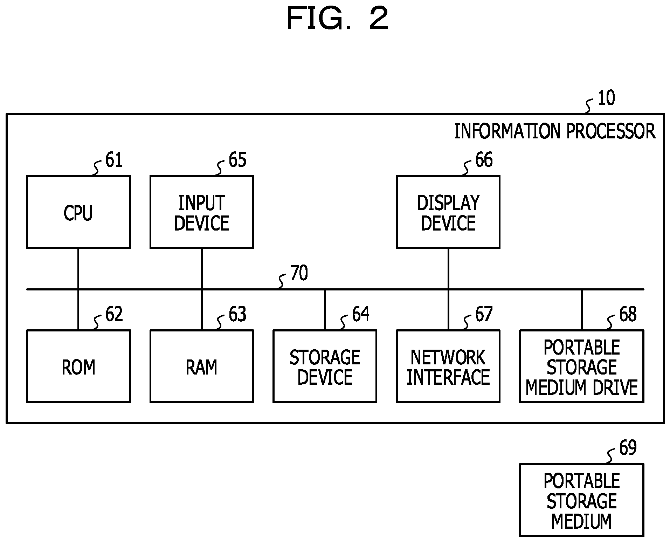

FIG. 2 is a diagram illustrating an example of the hardware configuration of the information processor 10 in Example 1. As illustrated in FIG. 2, the information processor 10 includes a CPU (Central Processing Unit) 61, a ROM 62, a RAM 63, a storage device 64, an input device 65, the display device 66, a network interface 67, a portable storage medium drive 68, and the like.

The CPU 61 is hardware that manages or executes the processing of the information processor 10. An MPU (Micro Processing Unit) is also an example of the CPU 61. The CPU 61 is an example of the reception unit 13, the feature amount calculation unit 14, the digestibility estimation unit 15, the determination unit 16, the correction unit 17, the information presentation unit 18, and the table generation unit 19.

The ROM 62, the RAM 63, and the storage device 64 are hardware that stores data and programs used for processing to be executed by the CPU 61. The storage device 64 is, for example, an HDD. The ROM 62, the RAM 63, and the storage device 64 are an example of the storage unit 12 illustrated in FIG. 1.

The input device 65 is hardware used by the user to input various information. The input device 65 is, for example, a touch panel, a keyboard, a mouse, and the like. When the input device 65 is a touch panel, the touch panel is placed so as to overlap the display device 66 to be described later. The input device 65 is an example of the input unit 11 illustrated in FIG. 1.

The display device 66 is a device that displays characters or images. The display device 66 may have a function to output voices. The display device 66 is, for example, a liquid crystal display, a plasma display, an organic EL (ElectroLuminescence) display, or the like.

The network interface 67 is hardware for communicating with an external device through a network.

The respective units included in the information processor 10 are connected to a bus 70. The information processor 10 has its functions realized by a processor such as the CPU 61 executing programs (including the digestibility estimation program) stored in the ROM 62 or the storage device 64 or programs (including the digestibility estimation program) read from a portable storage medium 69 by the portable storage medium drive 68. Note that such programs may be loaded into the RAM 63 and executed by the processor such as the CPU 61.

Next, description is given of a digestibility estimation method executed by the information processor 10. The inventor of the present invention has found out that there is a specific trend in a profile of changes in heart rate by measuring changes in heart rate between before meal and after meal.

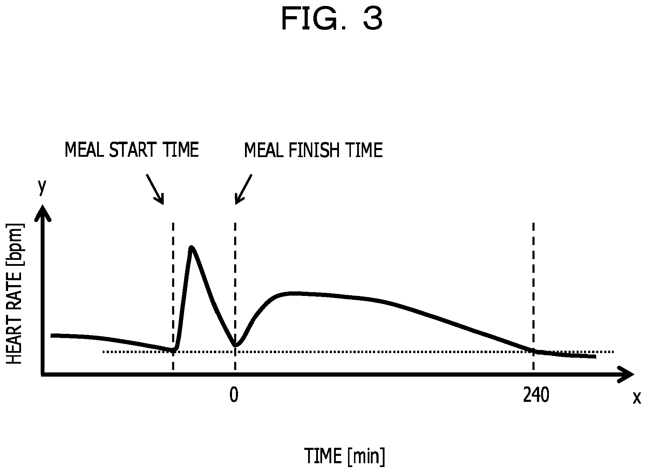

FIG. 3 is a graph illustrating an example of a temporal change in heart rate between before meal and after meal. The vertical axis (y-axis) represents a heart rate, while the horizontal axis (x-axis) represents a time axis with the meal finish time set as 0 minutes. The meal start time means the time when a meal is started, while the meal finish time means the time when the meal is finished. Note that, in the following description, a profile of temporal changes in heart rate may be called a "heart rate profile". As illustrated in FIG. 3, the heart rate rises rapidly as the meal is started, and then drops to a local minimum value at the meal finish time. This local minimum value is a level slightly higher than the heart rate at the meal start time. It is inferred that such changes in heart rate during a meal are attributable to chewing behavior or swallowing behavior during eating of food.

Thereafter, the heart rate rises again as the meal is finished, and gradually drops after reaching its peak at a certain time. Then, the heart rate returns to the one at the meal start time and eventually becomes fixed. It is inferred that such changes in heart rate after meal are attributable to a digestive activity in the stomach or the like in the body, and the heart rate returns to the level before meal as the digestive activity is finished. In the example of FIG. 3, the heart rate returns to the level before meal 240 minutes after the meal finish time. However, the time required for the heart rate to return to the level before meal varies depending on the content (kind, quantity, or the like) of the meal, eating speed, or the like. In this embodiment, the digestibility is estimated in real time by using the trend of changes in heart rate described above.

Next, description is given of a flow of processing executed by the information processor 10 to estimate the digestibility and present information to the user based on the estimation result.

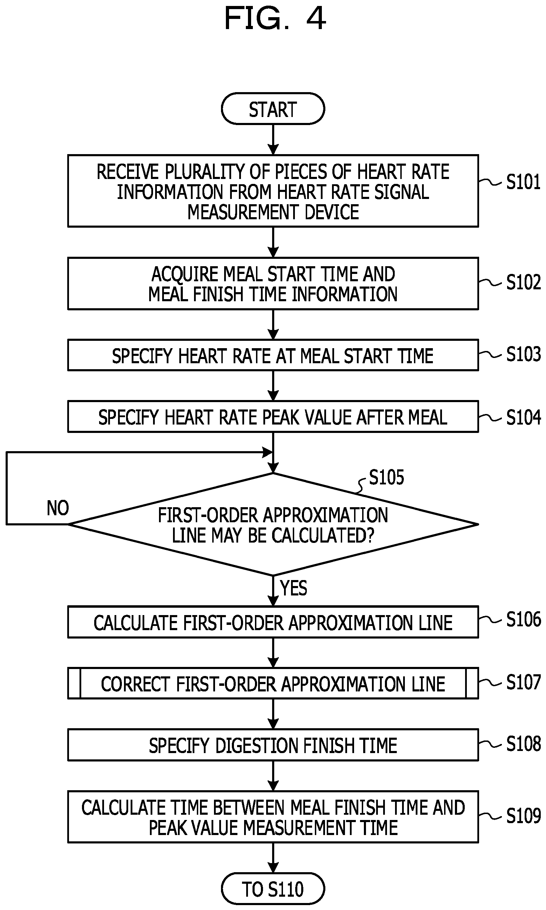

FIG. 4 is a flowchart (Part 1) illustrating an example of the digestibility estimation method executed by the information processor 10.

First, the first receiver 13a receives a plurality of pieces of heart rate signal information from the heart rate signal measurement device 20 (S101). In S101, the first receiver 13a acquires a plurality of pieces of heart rate signal information from the heart rate signal measurement device 20 at specified time intervals through wireless communication, for example. Then, the first receiver 13a stores the acquired plurality of pieces of heart rate signal information in the heart rate management table 12a in the storage unit 12.

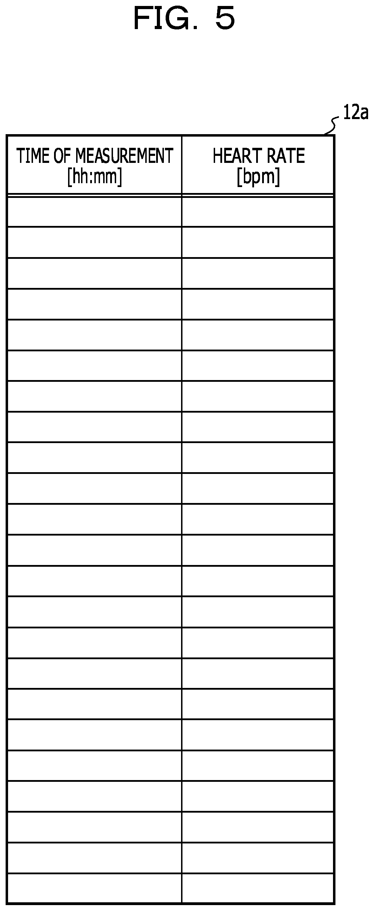

FIG. 5 is a diagram illustrating an example of an initial state of the heart rate management table 12a. The heart rate management table 12a has items of heart rate and time of measurement that is the time when the heart rate is measured.

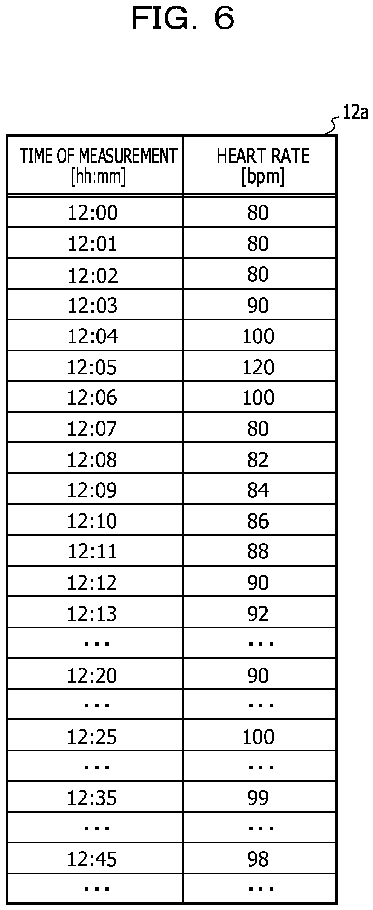

FIG. 6 is a diagram illustrating an example where information is stored in the heart rate management table 12a. In the example of FIG. 6, "12:00" means 12 o'clock, and information on heart rates measured at 1-minute intervals is stored. The information stored in the heart rate management table 12a is managed every day. For example, the measurement may be started at 0:00 every day and the information may be deleted every time the date changes. This method may suppress an increase in storage area used for the heart rate management table 12a in the storage unit 12.

Referring back to FIG. 4, after S101, the second receiver 13b acquires meal start time and meal finish time information when sequentially receiving the heart rate information from the heart rate signal measurement device 20 (S102). In S102, for example, the user uses the input unit 11 to input the meal start time and meal finish time information. Thus, the second receiver 13b may receive the meal start time and meal finish time information.

Alternatively, the information processor 10 may also use a method for specifying the meal start time and the meal finish time by detecting the movement of a lower arm during a meal with an acceleration sensor, as another method for acquiring the meal start time and meal finish time information (see Patent Literature 4). Alternatively, the information processor 10 may also use a method for specifying the meal start time and the meal finish time by detecting a chewing-specific frequency pattern with an intracorporeal sound microphone (see Patent Literature 5).

In S102, the second receiver 13b stores the acquired meal start time and meal finish time information in the meal time management table 12b in the storage unit 12.

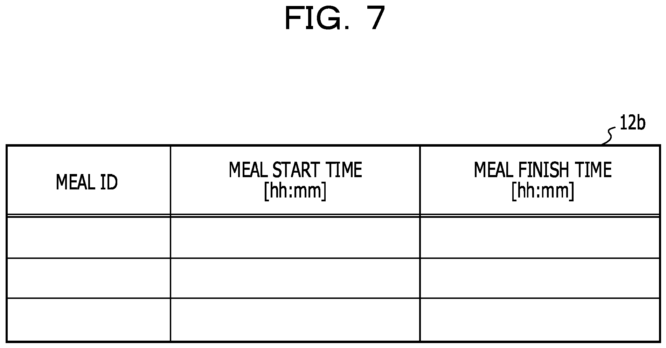

FIG. 7 is a diagram illustrating an example of an initial state of the meal time management table 12b. As illustrated in FIG. 7, the meal time management table 12b has items of meal ID (identifier) indicating an identifier to specify a meal event, meal start time, and meal finish time, and manages the meal start time and meal finish time information for each meal ID. The second receiver 13b provides a new meal ID to register in the meal time management table 12b upon every receipt of the meal start time and meal finish time information.

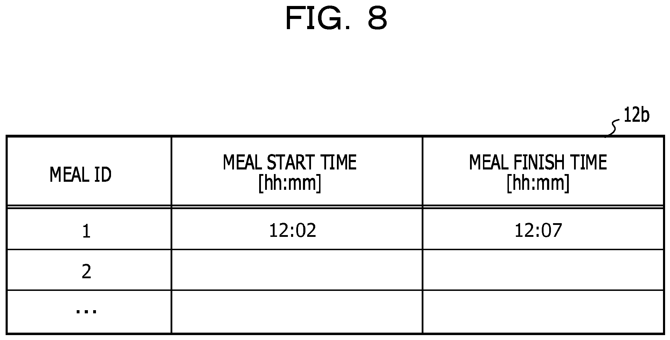

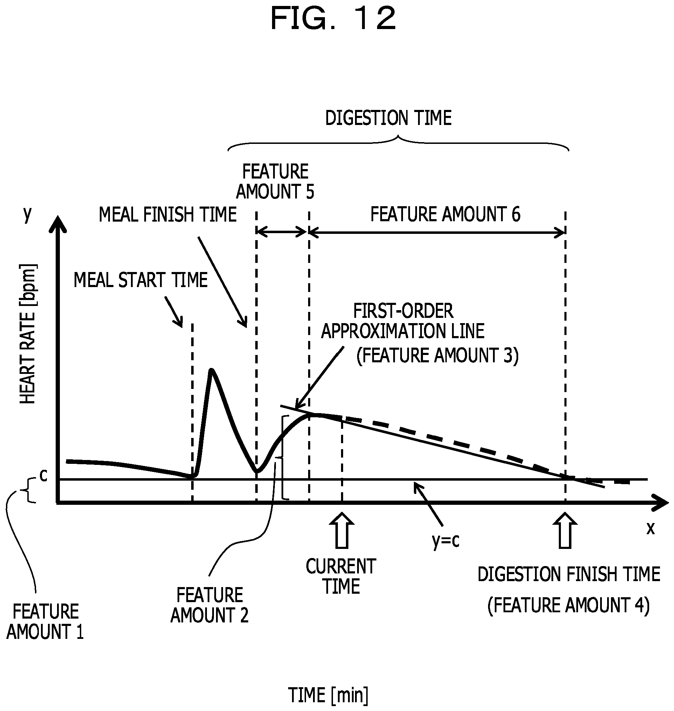

FIG. 8 is a diagram illustrating an example where information is stored in the meal time management table 12b in S102. In the example of FIG. 8, 12:02 is registered as the meal start time and 12:07 is registered as the meal finish time for a meal with the meal ID=1. With this information, a processing flow is described below.

After S102, the feature amount calculation unit 14 identifies a heart rate at the meal start time (S103). To be more specific, a heart rate associated with the meal start time registered in the meal time management table 12b of FIG. 8 is identified by referring to the heart rate management table 12a of FIG. 6. For example, FIG. 8 illustrates that the meal start time corresponding to the meal ID=1 is 12:02. Therefore, it may be seen from FIG. 6 that the heart rate corresponding to 12:02 is 80 bpm (beats per minute). Thus, the feature amount calculation unit 14 stores information of the identified heart rate 80 bpm in the feature amount management table 12c.

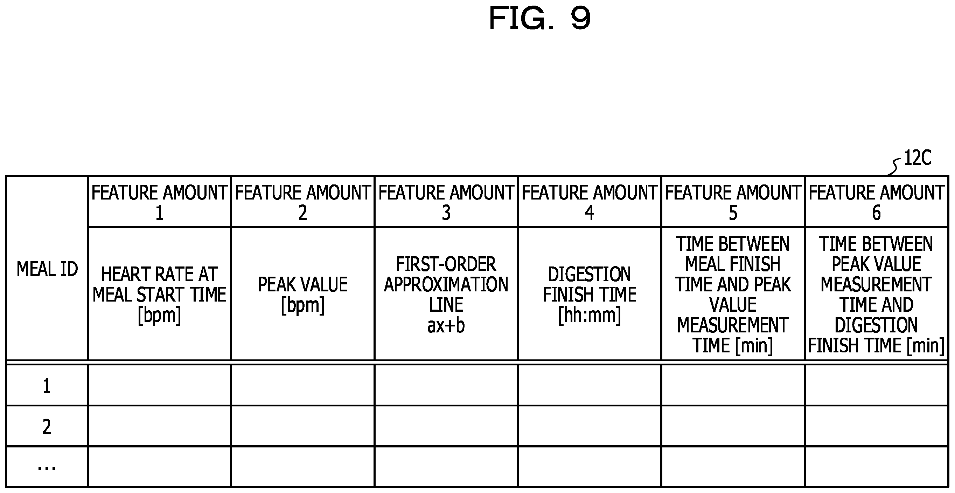

FIG. 9 is a diagram illustrating an example of an initial state of the feature amount management table 12c. As illustrated in FIG. 9, the feature amount management table 12c may store six kinds of feature amounts, Feature Amount 1 to Feature Amount 6 for each meal ID. In the description of this embodiment, the heart rate at the meal start time is referred to as "Feature 1" and a peak value indicating the maximum value of the heart rate after meal is referred to as "Feature Amount 2". Moreover, a first-order approximation line of a heart rate profile after the time of measurement of the peak value is referred to as "Feature Amount 3", and the digestion finish time is referred to as "Feature Amount 4". Furthermore, time between the meal finish time and the time of measurement of the peak value is referred to as "Feature Amount 5", and time between the time of measurement of the peak value and the digestion finish time is referred to as "Feature Amount 6". Note that Feature Amounts 2 to 6 are described in detail later.

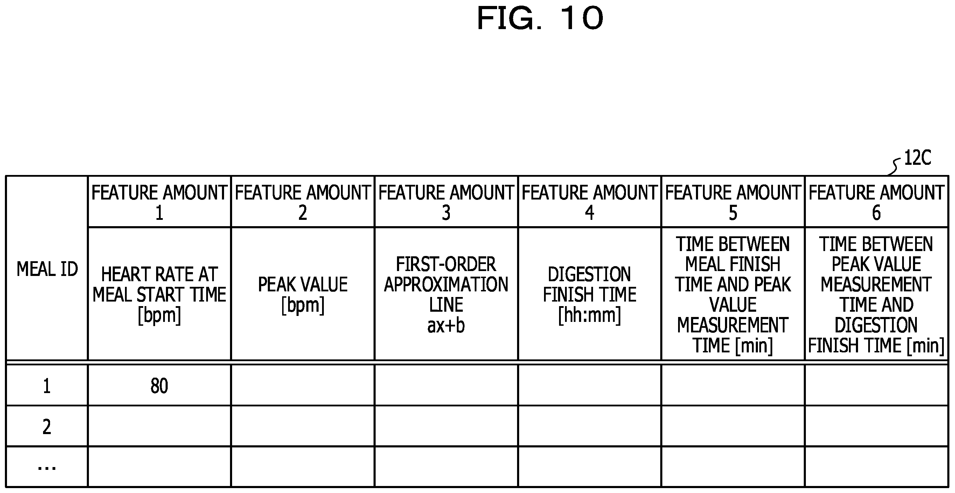

FIG. 10 is a diagram illustrating an example where information is stored in the feature amount management table 12c in S103. As illustrated in FIG. 10, the feature amount calculation unit 14 stores the information of the heart rate 80 bpm identified in S103 in the field of the item "Feature Amount 1" corresponding to the meal ID=1.

Subsequently, the feature amount calculation unit 14 identifies a peak value of the heart rate after meal (S104). For example, when the meal finish time is 12:07 as illustrated in FIGs. 8 and 6 illustrates a storage state of the heart rate management table 12a at the start of processing in S104, the feature amount calculation unit 14 refers to the heart rate management table 12a of FIG. 6. Then, the feature amount calculation unit 14 identifies that the peak value of the heart rate in a time slot after meal is 100 bpm measured at the time 12:25.

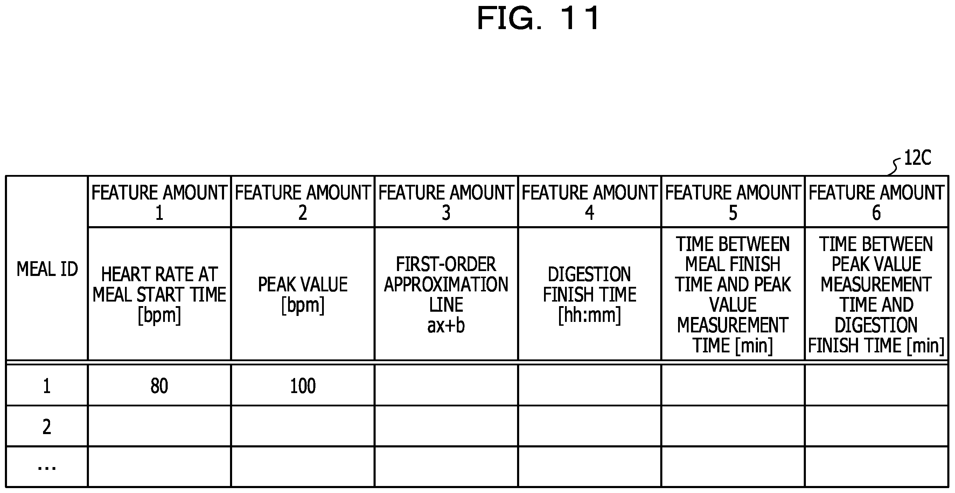

FIG. 11 is a diagram illustrating an example where information is stored in the feature amount management table 12c in S104. As illustrated in FIG. 11, the feature amount calculation unit 14 stores the identified peak value 100 bpm in the field of the item "Feature Amount 2" corresponding to the meal ID=1.

Then, the determination unit 16 determines whether or not a first-order approximation line may be calculated (S105). Here, description is given of the background of processing in S105.

As described with reference to FIG. 3, the heart rate profile has a shape in which the heart rate rises again after meal and gradually drops before returning to the heart rate at the meal start time after reaching its peak. However, during a digestive activity, the heart rate profile drawn with a plurality of pieces of heart rate information acquired up to that point has not yet reached the heart rate at the meal start time. Therefore, the time when the digestive activity is finished (digestion finish time) is yet to be identified. For this reason, in this embodiment, a first-order approximation line is used to identify the digestion finish time with the heart rate profile that has not reached the heart rate at the meal start time.

FIG. 12 is a diagram for explaining a method for estimating a digestibility with a first-order approximation line. In FIG. 12, a profile drawn with the heart rate information acquired by the first receiver 13a is indicated by the solid line. The vertical axis (y-axis) represents a heart rate, while the horizontal axis (x-axis) represents a time axis when the time of measurement of a peak value of the heart rate after meal is set as 0 minutes. Also, the right end of the profile indicated by the solid line represents the magnitude of the heart rate measured at the current time. Moreover, a profile drawn with unknown heart rate information yet to be acquired by the first receiver 13a is indicated by the broken line.

As illustrated in FIG. 12, the information processor 10 approximates, by a first-order approximation line, a change in heart rate in a predetermined time slot after the time of measurement of the peak value of the heart rate after meal. With the x-axis as the time axis and the y-axis as a coordinate axis indicating the magnitude of the heart rate, the first-order approximation line may be expressed as $y = ax + b$. Here, $a$ represents the slope of the heart rate profile indicating a downward trend after the time of measurement of the heart rate peak value. As described later, $b$ represents the heart rate at the time of measurement of the heart rate peak value. Assuming that the time when the heart rate returns to a value $c$ after meal is the digestion finish time, $c$ being the value of the heart rate at the meal start time, the digestion finish time may be calculated by obtaining an x-coordinate value at the intersection of $y = ax + b$ and $y = c$. It is assumed, in this embodiment, that a time slot of about 10 minutes to 20 minutes before the current time and after the time of measurement of the peak value, for example, is the "predetermined time slot" described above. When heart rate information in that time slot is acquired, the information processor 10 calculates a first-order approximation line by approximating a change in heart rate after the time of measurement of the peak value by a line. S105 is processing for determining whether or not the heart rate information required to calculate the first-order approximation line has been acquired already.

In S105, the determination unit 16 first sets 20 minutes, for example, as a setting time for the time that has elapsed since the time of measurement of the peak value.

Thereafter, the determination unit 16 determines, by referring to the heart rate management table 12a of FIG. 6, whether or not all the heart rate information in the time slot between the time of measurement of the peak value and 20 minutes later is stored. When not all the heart rate information is stored, the determination unit 16 determines that the first-order approximation line may not be calculated, since there is not enough heart rate information to calculate the first-order approximation line (S105: No). When the determination unit 16 determines that the first-order approximation line may not be calculated (S105: No), the determination unit 16 waits until 20 minutes pass after the time indicating the peak value and the heart rate information required to calculate the first-order approximation line is acquired. In other words, the determination unit 16 repeatedly executes the processing of S105.

On the other hand, when all the target heart rate information is stored, it means that the heart rate information required to calculate the first-order approximation line has been acquired. Therefore, the determination unit 16 determines that the first-order approximation line may be calculated (S105: Yes), and the feature amount calculation unit 14 calculates the first-order approximation line (S106). In S106, the feature amount calculation unit 14 extracts, from the heart rate management table 12a, a plurality of pieces of heart rate information in the predetermined time slot after the time of measurement of the peak value. Then, the feature amount calculation unit 14 uses the information of the peak value stored in the field of the item "Feature Amount 2" in the feature amount management table 12c and the extracted plurality of pieces of heart rate information to calculate the first-order approximation line that is a function of a heart rate with time as a variable.

As illustrated in FIG. 12, for example, assuming that the time of measurement of the heart rate peak value after meal is the origin on the time axis (x-axis), Feature Amount 2, that is, the heart rate peak value 100 bpm is the value at the y-intercept of the first-order approximation line. Meanwhile, with reference to the heart rate management table 12a of FIG. 6, the time of measurement of the peak value 100 bpm is 12:25. Therefore, when the setting time set in S105 is 20 minutes, the feature amount calculation unit 14 extracts a plurality of pieces of heart rate information up to 12:45 that is 20 minutes after 12:25. Then, the feature amount calculation unit 14 calculates the slope of the first-order approximation line of the heart rate, based on the extracted plurality of heart rate values, to obtain a value -0.1. As a result, assuming that the time that has elapsed since the time of measurement of the peak value is x minutes, the feature amount calculation unit 14 may obtain y= -0.1x+100 as the first-order approximation line of the heart rate.

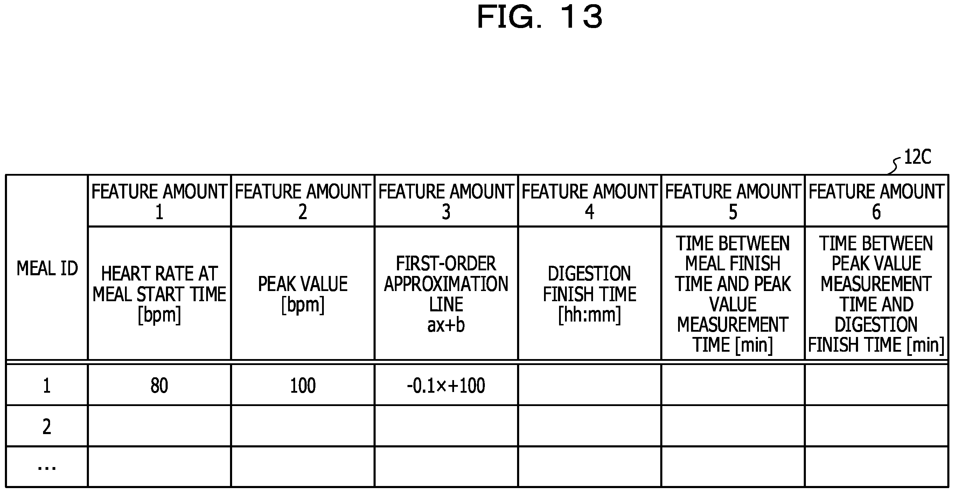

FIG. 13 is a diagram illustrating an example where information is stored in the feature amount management table 12c in S105. As illustrated in FIG. 13, the feature amount calculation unit 14 stores the information of the calculated first-order approximation line -0.1x+100 in the field of the item "Feature Amount 3" corresponding to the meal ID=1.

Referring back to FIG. 4, after S106, the correction unit 17 corrects the first-order approximation line acquired in S106 (S107). Here, description is given of the background of processing in S107.

The inventor of the present invention has found out that a profile of a change in heart rate to be obtained varies depending on an eating speed even with the same meal content. To be more specific, when the user eats a certain meal at a speed faster than a normal speed, in other words, performs so-called "speed-eating", the heart rate tends to be higher during meal and take longer to recover after meal. On the other hand, when the user eats the meal at a speed slower than a normal speed, in other words, performs so-called "slow-eating", the heart rate tends to be lower during meal and more quickly recover after meal.

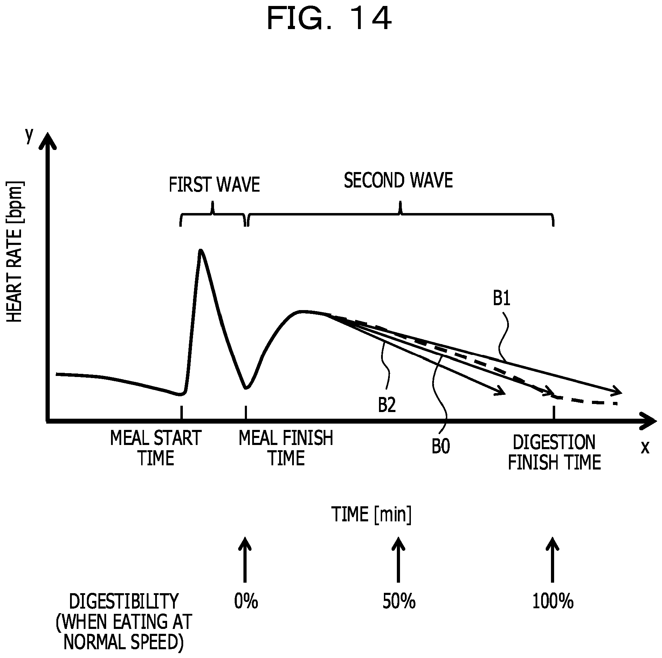

FIG. 14 is a diagram (Part 1) for explaining the background of the processing in S107. FIG. 14 illustrates a profile of temporal changes in heart rate when the user eats at a normal speed, which has the same trend as that of FIG. 12. A change in heart rate that rises with time and then drops between the meal start time and the meal finish time is defined as a "first wave", while a change in heart rate that rises with time and then drops between the meal finish time and the digestion finish time is defined as a "second wave". In FIG. 14, a vector B0 represents a vector overlapping with the first-order approximation line y= ax+b when the user eats at the normal speed.

When the user performs "speed-eating", it takes longer for the heart rate to recover after meal. Thus, the width of the second wave in the time axis direction is longer than that when the user eats at the normal speed. As a result, the direction of change in the second wave after its peak is closer to a vector B1 rather than the vector B0. On the other hand, when the user performs "slow-eating", the heart rate recovers more quickly after meal. Thus, the width of the second wave in the time axis direction is shorter than that when the user eats at the normal speed. As a result, the direction of change in the second wave after its peak is closer to a vector B2 rather than the vector B0.

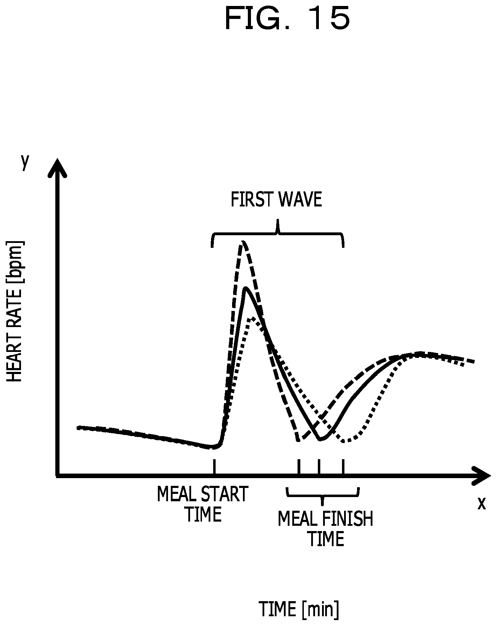

FIG. 15 is a diagram (Part 2) for explaining the background of the processing in S107. In FIG. 15, the solid line indicates an example of a first wave profile when the user eats at a normal speed, the broken line indicates an example of a first wave profile when the user performs "speed-eating", and the dotted line indicates an example of a first wave profile when the user performs "slow-eating".

As illustrated in FIG. 15, when the user performs "speed-eating", the meal time is shorter than that when the user eats at the normal speed. However, the heart rate during meal is higher than that when the user eats at the normal speed. On the other hand, when the user performs "slow-eating", the meal time is longer than the heart rate when the user eats at the normal speed. However, the heart rate during meal is lower than that when the user eats at the normal speed.

Given the above trends, in order to further improve digestibility prediction accuracy, it is desirable to take into consideration the eating speed. Therefore, in this embodiment, the first-order approximation line used to estimate a change in the second wave after its peak is corrected based on the trend of a change in the first wave. To be more specific, when the first wave indicates "speed-eating", the magnitude of the slope of the first-order approximation line is reduced to make a correction such that it takes longer for the digestibility to recover to 100% than when eating is performed at the normal speed. On the other hand, when the first wave indicates "slow-eating", the magnitude of the slope of the first-order approximation line is increased to make a correction such that the digestibility recovers more quickly to 100% than when eating is performed at the normal speed. Here, description is given of a specific example of processing in S107.

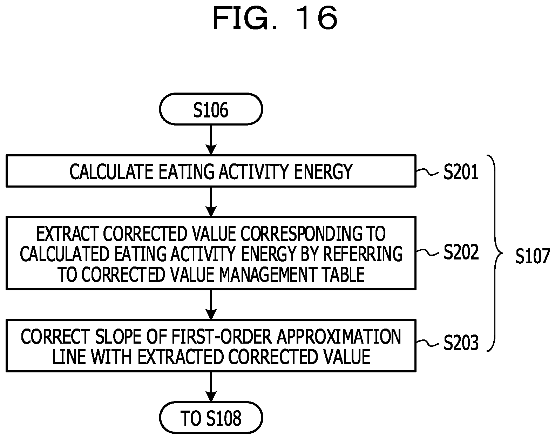

FIG. 16 is a flowchart illustrating a specific example of the processing in S107 in Example 1.

First, the feature amount calculation unit 14 uses a heart rate profile to calculate eating activity energy (S201). The eating activity energy is a feature amount indicating the level of energy of an eating activity. As for the eating activity energy, for example, an area of a figure extracted from the waveform of the first wave is calculated, and the value of the calculated area is used as an index value of the eating activity energy.

As a first example of calculating the area of the figure, for example, a method may be adopted wherein the shape of the first wave is approximated by a triangle and the area of the triangle contained in the first wave is calculated.

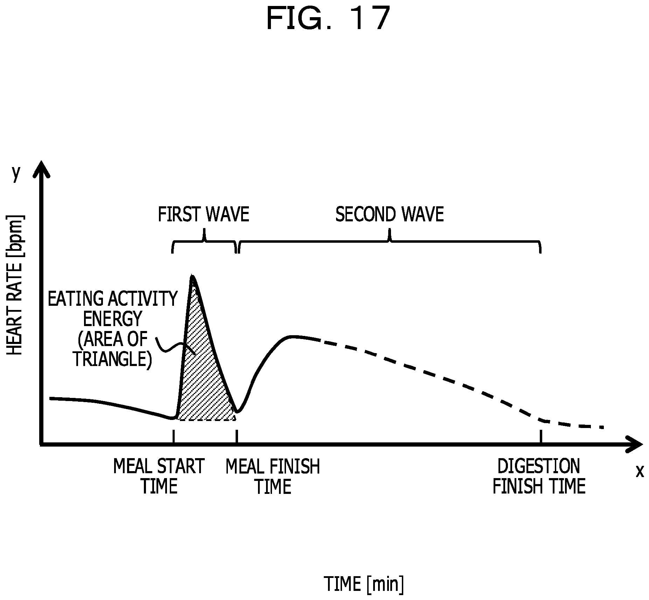

FIG. 17 is a diagram (Part 1) for explaining a method for calculating the area of the figure extracted from the waveform of the first wave. The figure indicated by the dotted line and shaded area in FIG. 17 represents the triangle contained in the first wave.

To be more specific, according to the meal time management table of FIG. 8, the meal start time corresponding to the meal ID=1 is 12:02, and the meal finish time is 12:07. Also, according to the heart rate management table of FIG. 6, the maximum heart rate during meal, that is, in a time slot between 12:02 and 12:07 is 120 bpm, and the heart rate at the meal start time is 80 bpm. Therefore, assuming that an amount of change in time is the base of the triangle and a difference between the maximum heart rate during meal and the heart rate at the meal start time is the height of the triangle, the eating activity energy may be calculated according to the following equation. Note that, since the unit of the eating activity energy is any unit, the notation of the unit of the eating activity energy may be omitted in the following description.

$$\text{(Eating Activity Energy)}$$

$$= [(\text{meal finish time-meal finish time}) \times (\text{maximum heart rate during meal-heart rate at meal start time})]/2$$

$$= 5\,[\text{min}] \times (120\text{-}80)\,[\text{bpm}]/2 = 100\,[\text{min/bpm}]$$

As a second example of calculating the area of the figure, for example, an area of a square containing the first wave may be calculated and the value of the calculated area may be used as an index value of the eating activity energy.

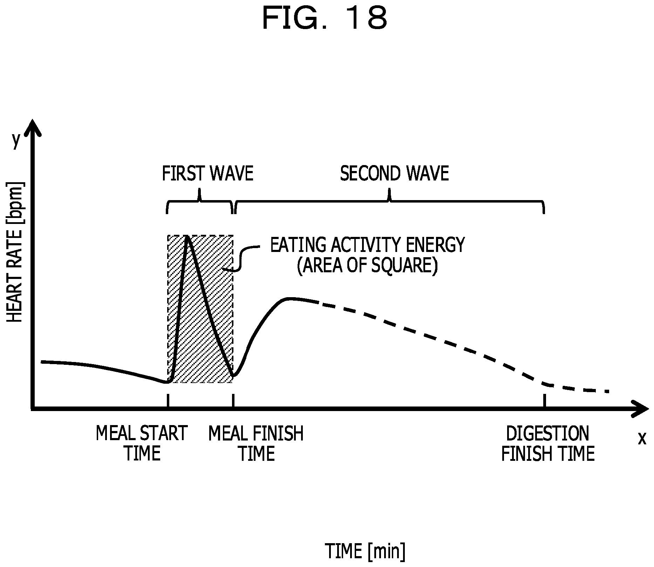

FIG. 18 is a diagram (Part 2) for explaining a method for calculating the area of the figure extracted from the waveform of the first wave. The figure indicated by the dotted line and shaded area in FIG. 18 represents the square containing the first wave. As for this square, an amount of change in time is set as the base, and the difference described above is set as the height. Therefore, the area of the square may be obtained by calculating the product of the amount of change in time and the difference between the maximum heart rate during meal and the heart rate at the meal start time. This method may improve the processing speed, since the method includes one less calculation step than the method for calculating the area of the triangle.

As a third example of calculating the area of the figure, for example, an area of a figure containing the first wave as a part of its contour may be calculated by time-integrating the first wave with a section between the meal start time and the meal finish time and the value (integrated value) of the calculated area may be used as an index value of the eating activity energy.

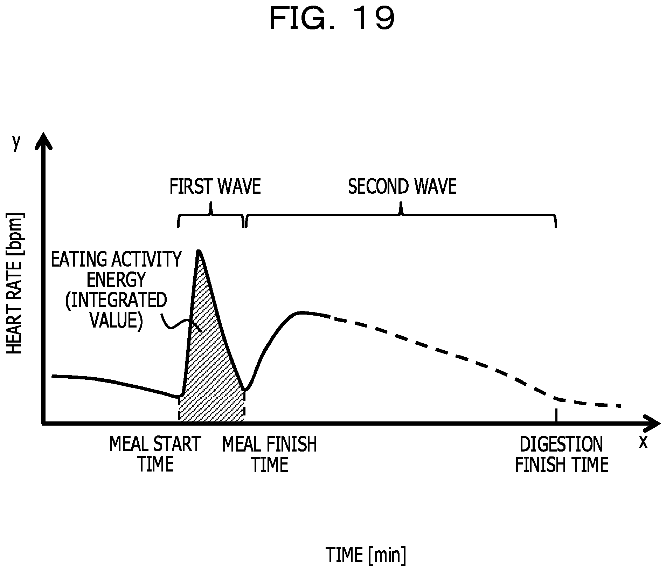

FIG. 19 is a diagram (Part 3) for explaining a method for calculating the area of the figure extracted from the waveform of the first wave. The figure indicated by the dotted line and shaded area in FIG. 19 represents a region subjected to time integration. The time integration may be performed, for example, by adding up all the heart rate values measured in the time slot between the meal start time and the meal finish time. According to this method, the eating activity energy may be calculated with higher accuracy compared with the approximation method with a triangle or a square, since the curve of the heart rate profile and the actual heart rate values are taken into consideration for the calculation of the area.

Meanwhile, as another example of calculating the eating activity energy, a rise rate when the heart rate rises with time during meal may be calculated and the value of the calculated rise rate may be used as an index value of the eating activity energy.

The first wave tends to be increased as the rise rate gets higher. According to this method, the eating activity energy may be calculated more easily than the method for calculating the area. Moreover, even when the shape of the figure containing the first wave as a part of its contour deviates from the triangle and approximation by the triangle becomes difficult, the eating activity energy may be calculated.

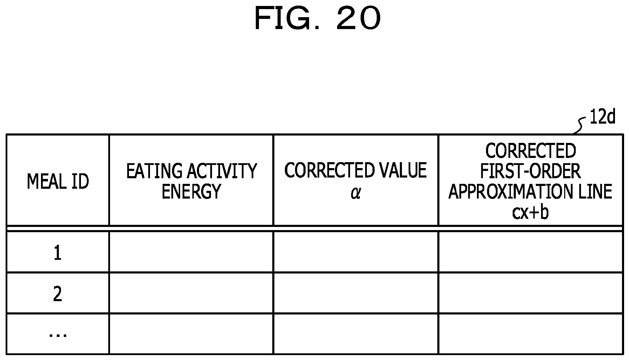

FIG. 20 is a diagram illustrating an example of an initial state of the first-order approximation line management table 12d. As illustrated in FIG. 20, the first-order approximation line management table 12d has items of meal ID, eating activity energy, corrected value $\alpha$, and first-order approximation line cx+b after correction. The first-order approximation line management table 12d stores information of the eating activity energy, corrected value $\alpha$, and the first-order approximation line (cx+b) after correction for each meal ID.

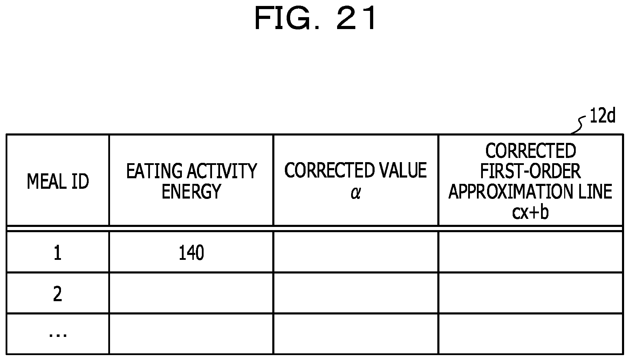

FIG. 21 is a diagram illustrating an example where information is stored in the first-order approximation line management table 12d in S201. As illustrated in FIG. 21, the feature amount calculation unit 14 stores the information of eating activity energy 140 calculated in S201 in the field of the item "eating activity energy" corresponding to the meal ID=1 as in the case of the feature amount management table 12c.

After S201, the correction unit 17 extracts a corrected value corresponding to the calculated eating activity energy by referring to the corrected value management table 12e (S202).

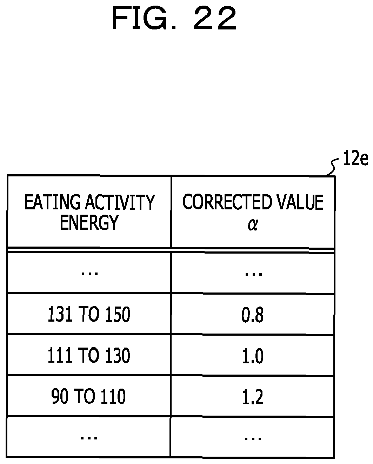

FIG. 22 is a diagram illustrating an example of the corrected value management table. As illustrated in FIG. 22, the corrected value management table 12e illustrates a correspondence relationship between a range of eating activity energy values and the corrected value $\alpha$ corresponding to the range. A method for generating the corrected value management table 12e is described later. In S202, when the eating activity energy value is 140, for example, it may be seen from the corrected value management table 12e that this value belongs to the range of eating activity energy values 131 to 150. Therefore, the correction unit 17 extracts 0.8 from the corrected value management table 12e as the corrected value $\alpha$ corresponding to that range.

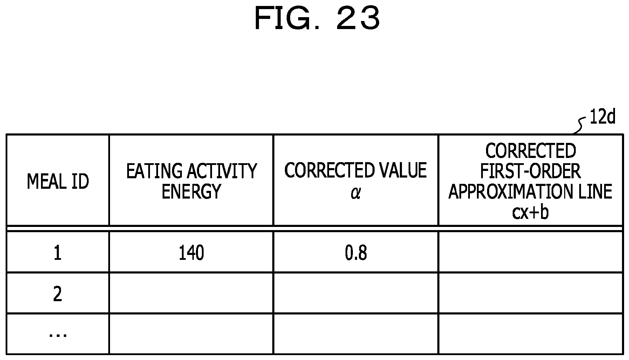

FIG. 23 is a diagram illustrating an example where information is stored in the first-order approximation line management table 12d in S202. As illustrated in FIG. 23, the feature amount calculation unit 14 stores the information of the corrected value $\alpha$= 0.8 calculated in S202 in the field of the item "corrected value" corresponding to the meal ID=1.

After S202, the correction unit 17 uses the corrected value $\alpha$ extracted in S202 to correct the slope of the first-order approximation line calculated in S106 (S203). To be more specific, the correction unit 17 corrects the slope of the first-order approximation line by multiplying the slope of the first-order approximation line by the corrected value $\alpha$. For example, the first-order approximation line is y= -0.1x+100 and the corrected value is $\alpha$= 0.8, the slope -0.1 of the first-order approximation line is multiplied by 0.8 to obtain a value -0.08. This value of the slope is applied to generate a new first-order approximation line y= -0.08x+100.

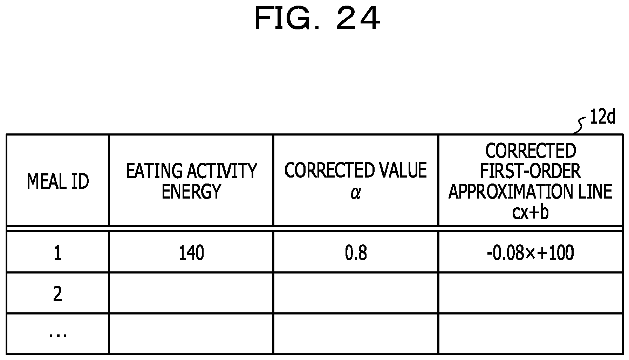

FIG. 24 is a diagram illustrating an example where information is stored in the first-order approximation line management table 12d in S203. As illustrated in FIG. 24, the feature amount calculation unit 14 stores the right-hand side information "-0.08x+100" of the first-order approximation line newly generated in S203 in the field of the item "first-order approximation line after correction" corresponding to the meal ID=1 in the first-order approximation line management table 12d.

The processing of S107 is thus executed. The processing moves to S108 after S107.

Referring back to FIG. 4, the feature amount calculation unit 14 uses the heart rate at the meal start time identified in S103 and the first-order approximation line corrected in S107 to calculate time between the peak value measurement time and the digestion finish time, and thus identify the digestion finish time (S108).

The feature amount calculation unit 14 uses the first-order approximation line obtained by approximating the profile when the heart rate drops to calculate the value x corresponding to the time when the heart rate returns to the one at the meal start time. With this method, the feature amount calculation unit 14 may identify the digestion finish time.

Referring to the item "Feature Amount 1" in the feature amount management table 12c of FIG. 13, the heart rate at the meal start time is 80 bpm. Therefore, the feature amount calculation unit 14 calculates the value x that satisfies 80= -0.08x+100 based on the corrected first-order approximation line stored in the first-order approximation line management table 12d. As a result, x= 250 is obtained, and the feature amount calculation unit 14 may specify that the digestive activity is completed 250 minutes after the peak value measurement time. The peak value measurement time is 12:25 according to the heart rate management table 12a of FIG. 6. Therefore, the feature amount calculation unit 14 calculates the digestion finish time as 250 minutes after 12:25, that is, 16:35.

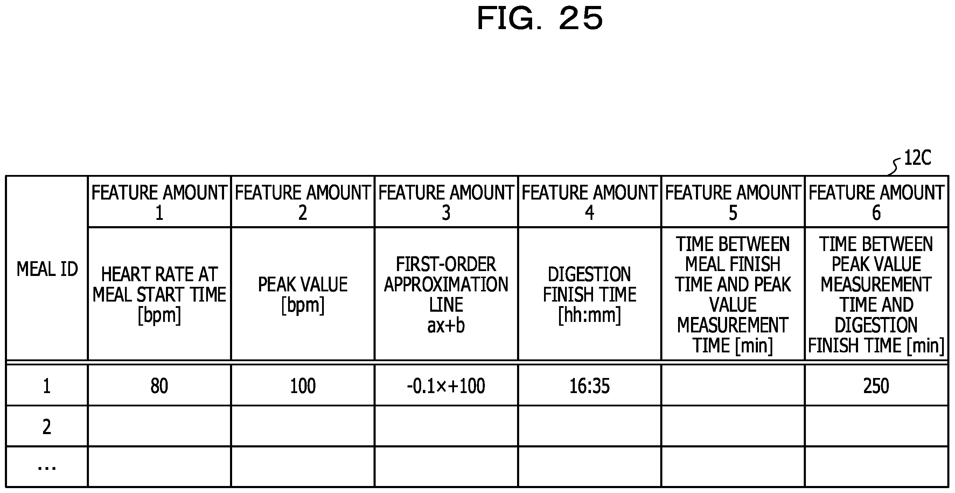

FIG. 25 is a diagram illustrating an example where information is stored in the feature amount management table 12c in S108. As illustrated in FIG. 25, the feature amount calculation unit 14 stores the information of the specified digestion finish time 16:35 in the field of the item "Feature Amount 4" corresponding to the meal ID=1. The feature amount calculation unit 14 further stores the value 250 minutes that is the time between the specified peak value measurement time and the digestion finish time in the field of the item "Feature Amount 6" corresponding to the meal ID=1. Thus, the feature amount calculation unit 14 may specify the digestion finish time at the end of the predetermined time slot by calculating the first-order approximation line with the heart rate information in the predetermined time slot after the peak value measurement time. As a result, the digestibility may be estimated in real time after the predetermined time slot.

Referring back to FIG. 4, after S108, the feature amount calculation unit 14 calculates time between the meal finish time and the peak value measurement time (S109). To be more specific, the feature amount calculation unit 14 calculates the time between the meal finish time and the peak value measurement time by obtaining a difference between the peak value measurement time and the meal finish time stored in the meal time management table 12b of FIG. 8. The time of measurement of the peak value (peak value measurement time) is 12:25 according to the heart rate management table 12a of FIG. 6. The meal finish time is 12:07 according to the meal time management table 12b of FIG. 8. Therefore, the time between the meal finish time and the peak value measurement time is calculated as 18 minutes.

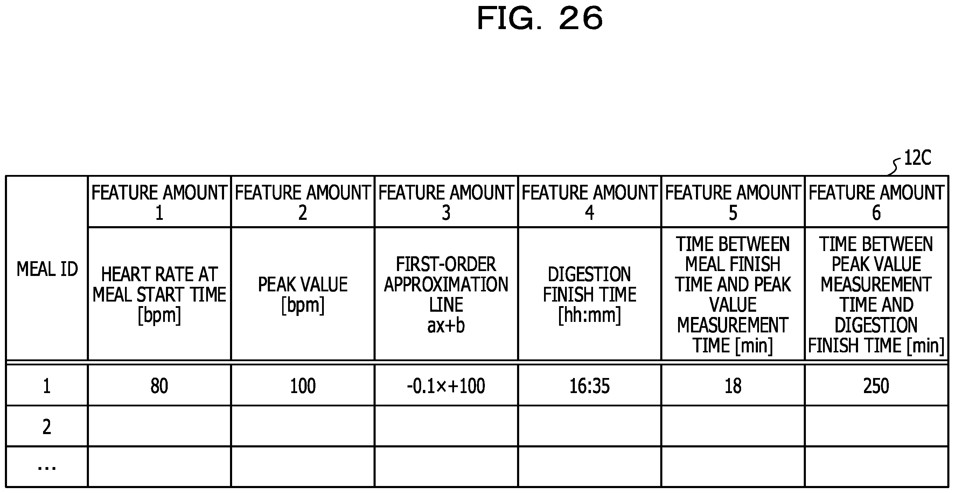

FIG. 26 is a diagram illustrating an example where information is stored in the feature amount management table 12c in S109. The feature amount calculation unit 14 stores the information of the specified time 18 minutes between the meal finish time and the peak value measurement time in the field of the item "Feature Amount 5" corresponding to the meal ID=1. The processing of S108 is thus executed. Hereinafter, processing to be executed after S109 is described.

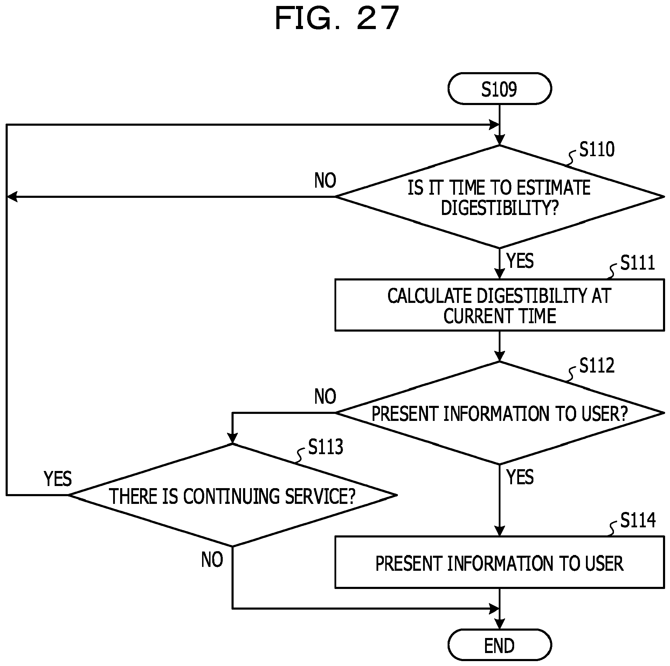

FIG. 27 is a flowchart (Part 2) illustrating an example of the digestibility estimation method executed by the information processor.

After S109, the determination unit 16 determines whether or not it is time to estimate the digestibility (S110). Since the digestibility is calculated at preset time intervals in this embodiment, the determination unit 16 executes the determination processing in S110. In the storage unit 12, the digestibility estimation management table 12f is prepared to store and manage the calculation result of the digestibility.

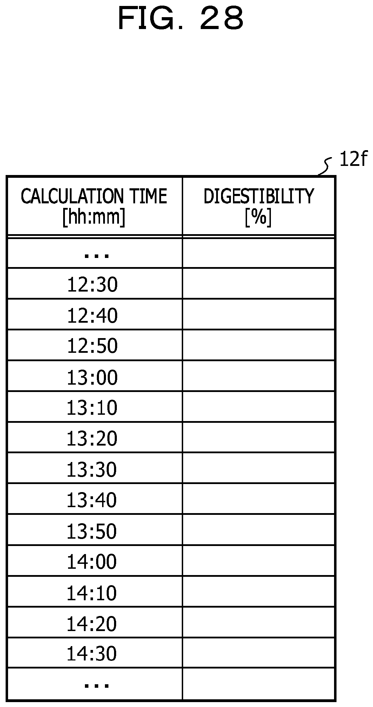

FIG. 28 is a diagram illustrating an example of an initial state of the digestibility estimation management table 12f. The digestibility estimation management table 12f has items of digestibility and calculation time indicating the time of calculation of the digestibility. In the example of FIG. 28, the time interval for the calculation of the digestibility is set to 10 minutes, and a plurality of calculation times are preset at 10-minute intervals. Note that the time interval may also be set to 1-minute or 1-second order.

Referring back to FIG. 27, in S110, the determination unit 16 determines whether or not it is time to estimate the digestibility by reading the current time information and then determining whether or not it is any one of the preset plurality of calculation times. When it is determined that it is not time to estimate the digestibility (S110: No), the processing of S110 is executed again. On the other hand, when it is determined that it is time to estimate the digestibility (S110: Yes), the digestibility estimation unit 15 calculates the digestibility at the current time (Sill).

On the assumption that the meal finish time is the time when the stomach starts its digestive activity (digestion start time) and the digestibility is 0% at the meal finish time and 100% at the digestion finish time, the digestibility estimation unit 15 calculates the digestibility at the current time based on the time that has elapsed since the meal finish time. To be more specific, the digestibility estimation unit 15 calculates the digestibility by calculating the length of time between the meal finish time and the digestion finish time, that is, a ratio of the length of time elapsed between the meal finish time and the current time to the digestion time.

The length of digestion time may be obtained by calculating the sum of "Feature Amount 5" and "Feature Amount 6" in the feature amount management table 12c as illustrated in FIG. 12. Thus, the digestibility at the current time is calculated according to the following equation.

$$(\text{Digestibility}) = [(\text{current time} - \text{meal finish time})/(\text{digestion time})] \times 100\%$$

$$= [(\text{current time} - \text{meal finish time})/(\text{Feature Amount 5} + \text{Feature Amount 6})] \times 100\%$$

It is assumed, for example, that the current time is 12:30. Since the meal finish time is 12:07 according to the meal time management table 12b of FIG. 8, a difference between the current time and the meal finish time is calculated as 23 minutes. According to the feature amount management table 12c of FIG. 26, "Feature Amount 5" is 18 minutes and "Feature Amount 6" is 250 minutes. Therefore, according to the above equation, the digestibility at the current time 12:30 is calculated as $(\text{Digestibility}) = [23/(18+250)] \times 100\% \approx 9\%$. The digestibility estimation unit 15 stores the value of the calculated digestibility 9% in the digestibility estimation management table 12f in association with the calculation time.

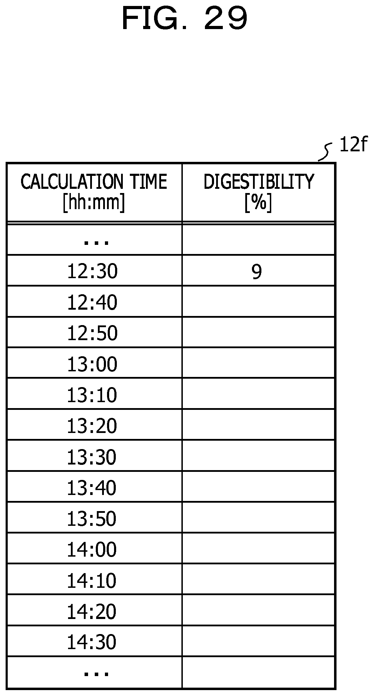

FIG. 29 is a diagram illustrating an example where information is stored in the digestibility estimation management table 12f in S111. As illustrated in FIG. 29, for example, the calculated value 9% is stored in a position corresponding to the calculation time 12:30 in the item of digestibility.

Then, the determination unit 16 determines whether or not to present service-related information to the user of the information processor 10, based on the calculated digestibility value (S112). Examples of the service may include a service of presenting and recommending restaurant such as a recommended restaurant when the user gets hungry and a service of giving advice to a user on a diet by presenting an appropriate time for eating. Examples of the service may also include a service of encouraging a user worried about his/her digestive capacity to have a meal when digestion is or is about to be completed and a service of giving an older user advice on appropriate time for eating. It is determined in S112 that the service-related information is presented to the user when the digestibility reaches a predetermined digestibility. The predetermined digestibility is within a range of, for example, 95% to 100%, which varies depending on the content of the service.

When it is determined that the service-related information is not to be presented to the user (S112: No), the determination unit 16 determines whether or not there is a continuing service to the user (S113). In S113, the determination unit 16 determines whether or not there is a continuing service to the user by determining, for example, whether or not the information processor 10 keeps on using an application related to the service. When it is determined that there is a continuing service to the user (S113: Yes), the processing returns to S110 to execute again the processing of S110 and thereafter. When it is determined that there is no continuing service to the user (S113: No), a series of processing is terminated.

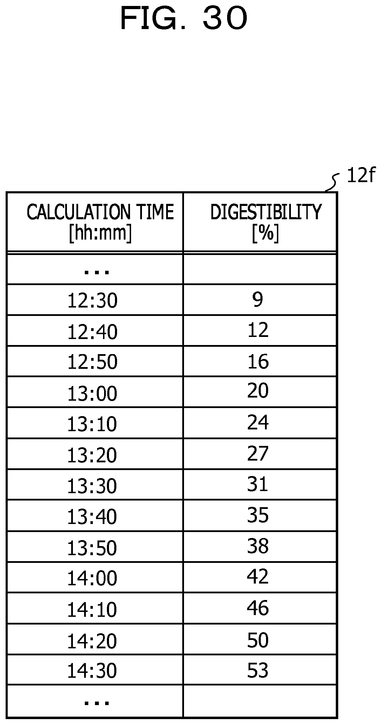

FIG. 30 is a diagram illustrating an example where information is stored more than once in the digestibility estimation management table 12f. As illustrated in FIG. 30, the processing of S112 is repeatedly executed until the result of the determination in S112 is Yes, that is, it is determined that the information is to be presented to the user. Then, the digestibility is calculated every 10 minutes, and the calculation results are gradually accumulated in the digestibility estimation management table 12f. The digestibility is 53% at 14:30, and the calculation of the digestibility and the accumulation of the results thereof are repeated until a predetermined digestibility is obtained.

When it is determined in S111 that the service-related information is to be presented to the user (S112: Yes), the information presentation unit 18 uses the display device 66 to present the information to the user (S114). When the information to be presented to the user includes information on a store such as a restaurant or a facility, and the like, the information to be presented to the user is acquired from an external device such as a server having a database (DB) that stores related information through a network, for example. Another method may also be adopted wherein the DB that stores related information is stored in the storage unit 12 in the information processor 10 and required information is read from the storage unit 12. After S114, a series of processing is terminated.

The digestibility estimation processing is thus executed by the information processor 10.

As described above, according to this embodiment, the information processor 10 specifies the time when the heart rate returns to the one at the meal start time after meal, as the digestion finish time, based on the trend of the heart rate that rises with time and then drops after meal and the heart rate at the meal start time, which are indicated by the plurality of pieces of heart rate information received from the heart rate signal measurement device 20. Then, the information processor 10 estimates the digestibility at the current time based on the information of the meal finish time acquired by the second receiver 13b and the digestion finish time. According to this method, the latest digestibility may be estimated based on the sequentially received latest heart rate information. Thus, services corresponding to the estimated digestibility may be provided to the user in real time. For example, when the user is receiving a service of providing information on restaurants corresponding to the digestibility, the user may be spared the trouble of searching for such restaurants.

Next, description is given of a method for generating the corrected value management table 12e used for the processing in S107.

In this embodiment, before execution of the processing of estimating the digestibility, processing of generating the corrected value management table 12e used to correct the first-order approximation line and storing the table in the storage unit 12 is performed as preprocessing.

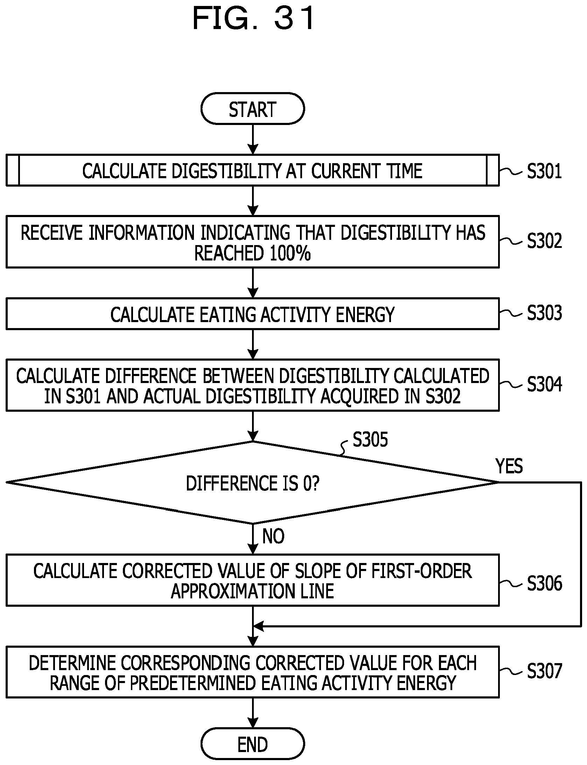

FIG. 31 is a flowchart illustrating an example of the method for generating a corrected value management table.

First, the digestibility estimation unit 15 calculates a digestibility at the current time according to the flowchart from S101 to S111 illustrated in FIGs. 4 and 27 (S301).

Then, when instinctively determining that the digestibility has reached 100%, the user inputs information to the input unit 11 to notify that the digestibility has reached 100%. Thereafter, the second receiver 13b receives the information from the input unit 11 (S302). The received information includes information on the time of the input performed by the user. The digestibility at that time is assumed to be 100% in this embodiment. Note that the user waits without inputting any information until he/she determines that the digestibility has reached 100%.

Subsequently, the feature amount calculation unit 14 calculates eating activity energy (S303). To be more specific, the feature amount calculation unit 14 uses the heart rate profile acquired during the processing of S301 to calculate the eating activity energy in the same manner as the processing of S201 in FIG. 16.

Then, the correction unit 17 calculates a difference between the digestibility calculated in S301 and the actual digestibility acquired in S302 (S304).

Thereafter, the determination unit 16 determines whether or not the difference in digestibility calculated in S304 is zero (S305). The processing moves to S307 when it is determined that the difference in digestibility is zero (S305: Yes). The processing of S307 is described later. On the other hand, when it is determined that the difference in digestibility is not zero (S305: No), the correction unit 17 calculates a corrected value of the slope of the first-order approximation line (S306). To be more specific, the correction unit 17 first specifies a point on the heart rate profile acquired in the processing of S301 at the time of measurement of the heart rate peak value after meal. Then, the correction unit 17 calculates a first-order approximation line that passes through the specified point and a point at a position corresponding to the heart rate of the meal finish time at the current time. This first-order approximation line is the actual first-order approximation line calculated based on the user's sense. Thereafter, the correction unit 17 calculates a corrected value $\alpha$ of the slope by dividing the slope of the calculated first-order approximation line by the slope of the first-order approximation line estimated in S301.

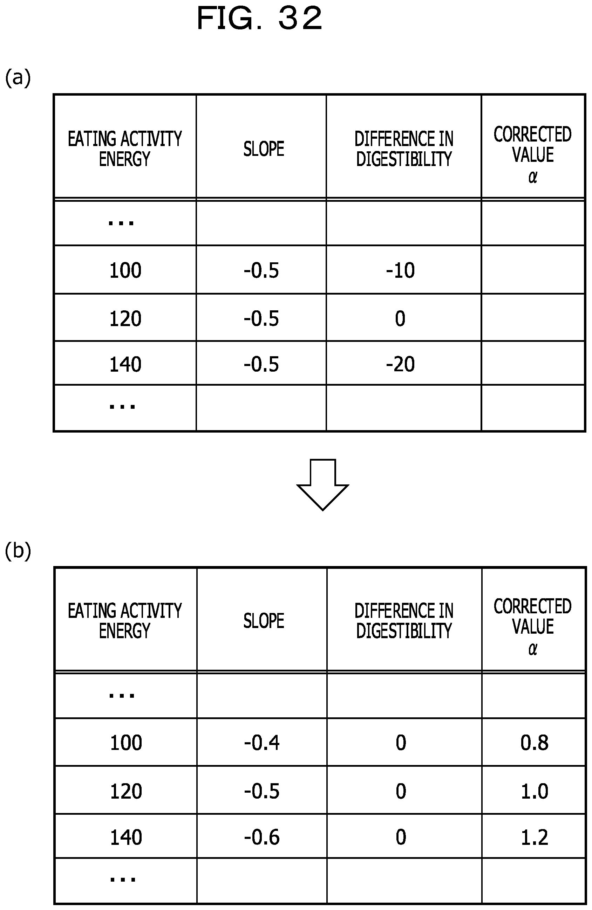

FIG. 32 is diagram illustrating a calculation example of the corrected value $\alpha$. FIG. 32(a) illustrates the slope of the first-order approximation line calculated in S301 and a difference between the digestibility 100% and the digestibility calculated in S301 for each of the calculated eating activity energy. As illustrated in FIG. 32(a), when the eating activity energy is 120, there is no difference. This means that the calculated digestibility corresponds to the actual digestibility. It may be seen that, on the other hand, there are differences in digestibility when the eating activity energy is 100 and 140. This means that the calculated digestibility does not correspond to the actual digestibility. FIG. 32(b) illustrates the slope of the actual first-order approximation line and the corrected value $\alpha$ calculated by dividing the slope of the actual first-order approximation line by the slope of the first-order approximation line calculated in S301. As illustrated in FIG. 32(b), in the case of the eating activity energy 120 where there is no difference in FIG. 32(a), the corrected value $\alpha$ is calculated as 1.0 and the result of the determination in S305 is Yes. On the other hand, in the case of the eating activity energy 100 and 140 where there are differences, the corrected value $\alpha$ is calculated as 0.8 and 1.2, respectively, and the result of the determination in S305 is No. In this way, a plurality of pieces of data associating the eating activity energy with the corrected value $\alpha$ may be obtained by calculating the corrected value $\alpha$ leading to zero difference by correction for each eating activity energy value.

After S306, the table generation unit 19 determines a corresponding corrected value for each range of predetermined eating activity energy, based on the accumulated plurality of pieces of data (S307). Through this processing, the table generation unit 19 may generate the corrected value management table as illustrated in FIG. 22.

Note that, although the corrected values $\alpha$ are calculated for the three eating activity energy values in the example of FIG. 32, the more the data, the more preferable. The table generation unit 19 may generate a more accurate corrected value management table with more data.

The table generation unit 19 may generate a corrected value management table based on information acquired from a plurality of users, for example, and allow the generated table to be shared by the plurality of users. With this method, the amount of the data described above may be easily increased. Thus, as in the above case, the accuracy of correcting the slope of the first-order approximation line may be improved.

Meanwhile, in general, there is an individual difference in digestive capacity and heart rate profile. Therefore, the table generation unit 19 may also use a method for generating a corrected value management table that differs for each user. This method makes it possible to rule out data obtained from a user having different digestive capacity and heart rate profile. Thus, the accuracy of correcting the slope of the first-order approximation line may be improved.

Furthermore, the table generation unit 19 may also classify a plurality of users into a plurality of groups based on, for example, age, heart rate, or a combination thereof, and generate a corrected value management table for each of the plurality of groups. With this method, one corrected value management table is generated by using data obtained from more than one user of the same type with a similar trend of heart rate profile. According to this method, not only data obtained from a user having a different trend of heart rate profile may be ruled out, but also the amount of data may be more easily increased compared with the method for generating a corrected value management table that differs for each user. With this method, again, the accuracy of correcting the slope of the first-order approximation line may be improved.

According to Example 1, the estimated time at which the heart rate returns to the one at the start of meal may be approximated to the time at which the heart rate actually returns to the one at the start of meal by correcting the slope of the first-order approximation line based on the heart rate profile during meal. Thus, a more accurate digestibility may be calculated and the accuracy of estimating the digestibility may be improved.

(EXAMPLE 2)

Next, Example 2 is described. Example 1 is characterized in that the slope of the first-order approximation line is corrected based on the heart rate profile during meal. On the other hand, Example 2 is characterized in that a value at the y-intercept of a first-order approximation line is corrected based on a heart rate profile during meal.

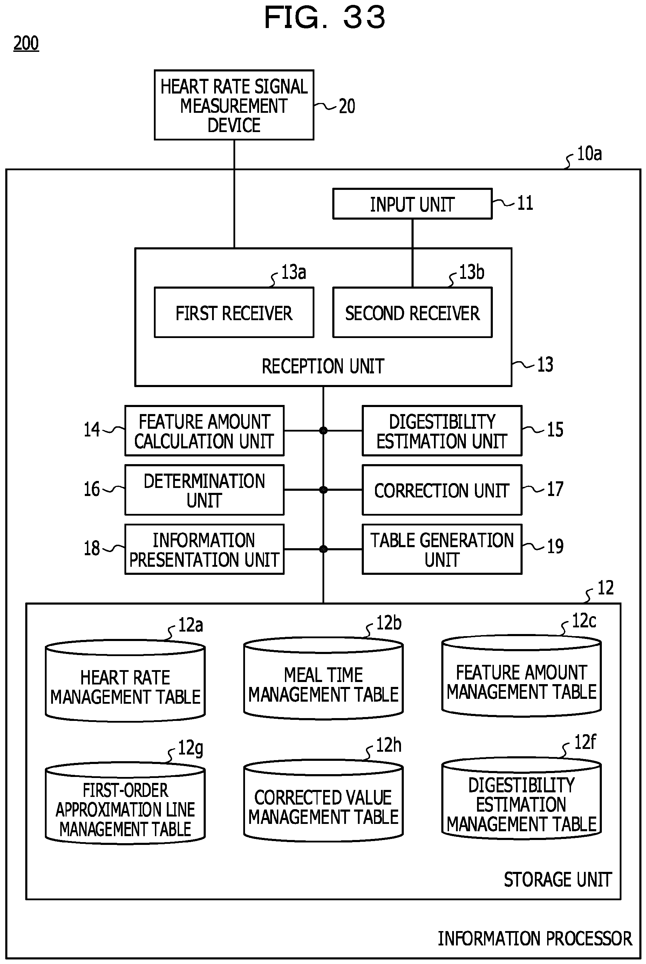

FIG. 33 is a diagram illustrating an example of an information processing system 200 in Example 2. The same functional blocks having the same functions as those of the functional blocks illustrated in FIG. 1 are denoted by the same reference numerals of those in FIG. 1, and description thereof is omitted. As illustrated in FIG. 33, a storage unit 12 in the information processing system 200 stores a first-order approximation line management table 12g and a corrected value management table 12h. Note that a hardware configuration diagram of an information processor 10a is the same as that in Example 1, and thus description thereof is omitted.

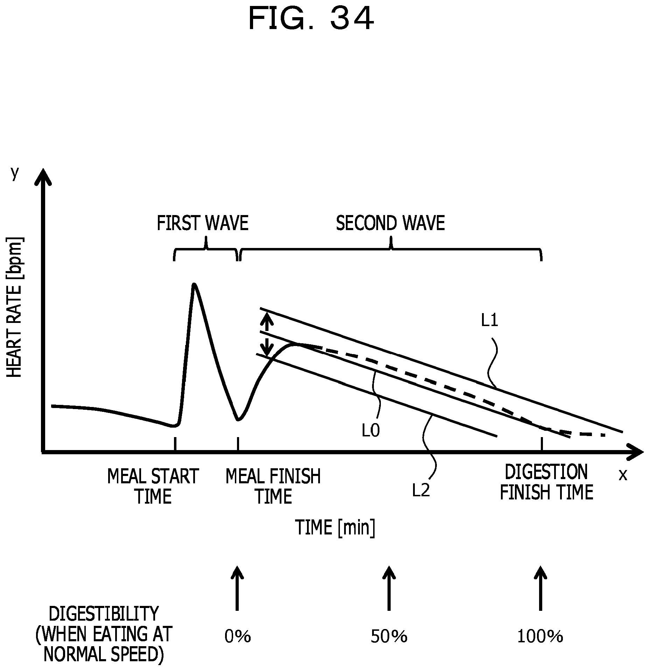

FIG. 34 is a diagram for explaining a method for correcting a first-order approximation line in Example 2. FIG. 34 illustrates a profile of temporal changes in heart rate having the same trend as that illustrated in FIG. 12. A line L0 represents a first-order approximation line y= ax+d when a user eats at a normal speed.

When the user performs "speed-eating", it takes longer for the heart rate to recover after meal. Thus, the width of the second wave in the time axis direction is longer than that when the user eats at the normal speed, and the digestion finish time is delayed. Therefore, the first-order approximation line is shifted from L0 to L1 by increasing the value at the y-intercept of the first-order approximation line. Accordingly, the estimated digestion finish time is shifted backward, and thus the first-order approximation line may be approximated to a line passing through a heart rate value at the actual digestion finish time.

On the other hand, when the user performs "slow-eating", the heart rate recovers more quickly after meal. Thus, the width of the second wave in the time axis direction is shorter than that when the user eats at the normal speed, and the digestion finish time is put forward. Therefore, the first-order approximation line is shifted from L0 to L2 by reducing the value at the y-intercept of the first-order approximation line. Accordingly, the estimated digestion finish time is shifted forward, and thus the first-order approximation line may be approximated to a line passing through a heart rate value at the actual digestion finish time.

Therefore, in this embodiment, the y-intercept of the first-order approximation line is corrected based on the trend of a change in the first wave. To be more specific, when the first wave indicates "speed-eating", the time required for the digestibility to reach 100% is corrected to be longer than that when eating is performed at the normal speed, by increasing the value at the y-intercept of the first-order approximation line. On the other hand, when the first wave indicates "slow-eating", the time required for the digestibility to reach 100% is corrected to be shorter than that when eating is performed at the normal speed, by reducing the value at the y-intercept of the first-order approximation line.

Next, description is given of a flow of processing executed by the information processor 10 in Example 2. A processing flowchart in Example 2 is approximately the same as that in Example 1 illustrated in FIG. 4. However, in Example 2, the content of processing in S107 is different from that in Example 1.

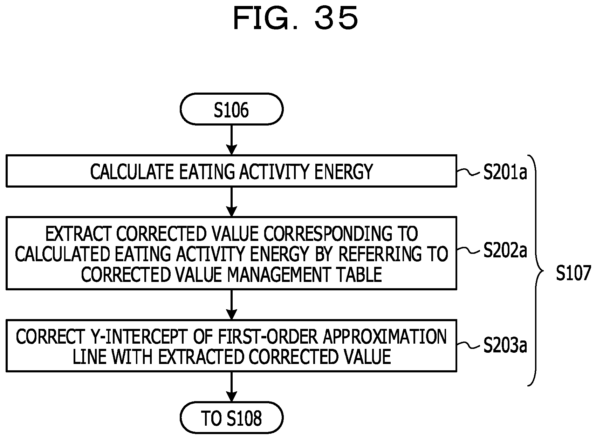

FIG. 35 is a flowchart illustrating a specific example of processing in S107 in Example 2.

First, the feature amount calculation unit 14 uses a heart rate profile to calculate eating activity energy (S201a). The processing in S201 is the same as that in S201 in Example 1.

Then, the correction unit 17 extracts a corrected value corresponding to the calculated eating activity energy by referring to the corrected value management table 12h (S202a).

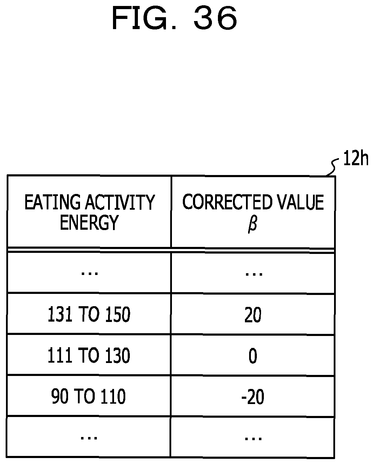

FIG. 36 is a diagram illustrating an example of the corrected value management table in Example 2. As illustrated in FIG. 36, the corrected value management table 12h of Example 2 illustrates a relationship between a range of eating activity energy values and a corrected value $\beta$ corresponding to the range. In S202a, when the eating activity energy value is 140, for example, it may be seen from the corrected value management table 12h that this value belongs to the range of eating activity energy values 131 to 150. Therefore, the correction unit 17 extracts 20 as the corrected value $\beta$ corresponding to that range. Note that, in the corrected value management table 12h, a relationship between the range of eating activity energy values and the corrected value $\beta$ may be set by narrowing the range of the eating activity energy values compared with the table illustrated in FIG. 36.

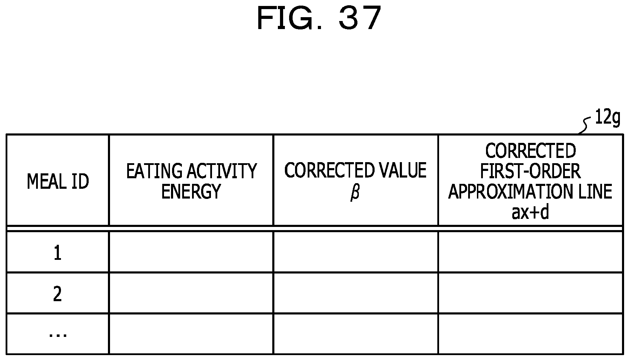

FIG. 37 is a diagram illustrating an example of an initial state of the first-order approximation line management table 12g. As illustrated in FIG. 37, the first-order approximation line management table 12g has items of meal ID, eating activity energy, corrected value $\beta$, and corrected first-order approximation line ax+d. The first-order approximation line management table 12g stores information of the eating activity energy, the corrected value $\beta$, and the corrected first-order approximation line ax+d for each meal ID.

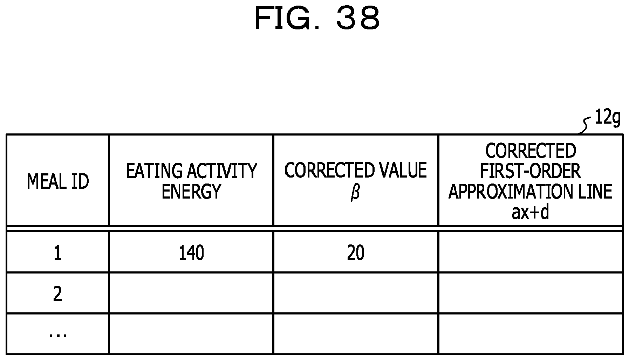

FIG. 38 is a diagram illustrating an example where information is stored in the first-order approximation line management table 12g in S202a. As illustrated in FIG. 38, the correction unit 17 stores the corrected value $\beta$= 20 extracted in S202a in the first-order approximation line management table 12g.

Then, the correction unit 17 uses the corrected value $\beta$ extracted in S202a to correct the y-intercept d of the first-order approximation line calculated in S106 (S203a). To be more specific, the correction unit 17 corrects the y-intercept d of the first-order approximation line by adding the corrected value $\beta$ to the value at the y-intercept of the first-order approximation line. For example, when the first-order approximation line is y= -0.1x+100 and $\beta$=20 is extracted as the corrected value, the value 120 is obtained by adding 20 to the y-intercept 100 of the first-order approximation line. This value of the y-intercept is applied to generate a new first-order approximation line y= -0.1x+120.

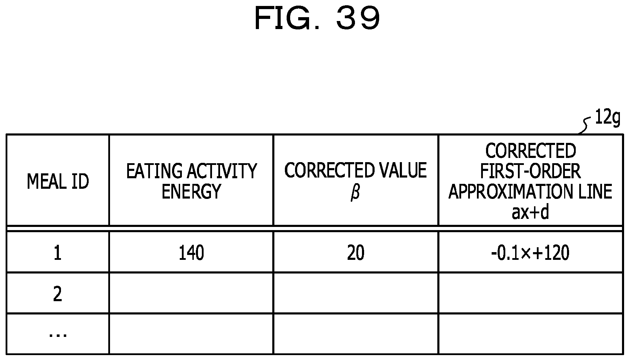

FIG. 39 is a diagram illustrating an example where information is stored in the first-order approximation line management table 12g in S203a. As illustrated in FIG. 39, the correction unit 17 stores the right-hand side information "-0.1x+120" of the first-order approximation line newly generated in S203a in the field of the item "corrected first-order approximation line ax+d" corresponding to the meal ID=1 in the first-order approximation line management table 12g

The processing of S107 is thus executed. The processing of S108 illustrated in FIG. 4 is executed after S107. The processing of S108 and thereafter is the same as that in Example 1, and thus description thereof is omitted.

According to Example 2, the estimated time at which the heart rate returns to the one at the start of meal may be approximated to the time at which the heart rate actually returns to the one at the start of meal by correcting the y-intercept of the first-order approximation line based on the trend of changes in heart rate profile during meal. Thus, a more accurate digestibility may be calculated and the accuracy of estimating the digestibility may be improved.

(EXAMPLE 3)

Next, Example 3 is described. In Examples 1 and 2, the information processor carried by the user receiving a service corresponding to the digestibility executes the processing of estimating the digestibility. On the other hand, Example 3 is characterized in that another information processor different from an information processor carried by a user executes processing of estimating a digestibility.

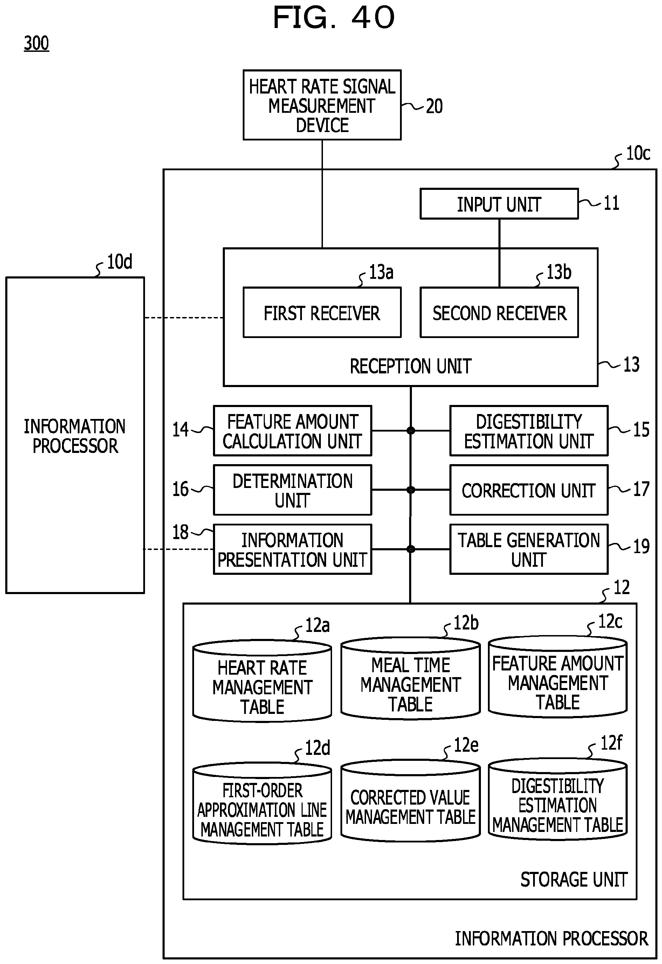

FIG. 40 is a diagram illustrating an example of an information processing system 300 in Example 3. In FIG. 40, the same functional blocks having the same functions as those of the functional blocks illustrated in FIG. 1 are denoted by the same reference numerals of those in FIG. 1, and description thereof is omitted. As illustrated in FIG. 40, the information processing system 300 includes an information processor 10b, an information processor 10c, and a heart rate signal measurement device 20. The broken lines in FIG. 40 represent wireless communication.

The information processor 10b is carried by the user, as in the case of the information processor 10 in FIG. 1. The information processor 10b is, for example, a computer such as a smartphone, a cell-phone, a tablet terminal, a laptop PC, and a wearable computer.

The information processor 10c is another information processor different from the information processor 10b, which estimates a digestibility of the user and is installed in a place away from the user. The information processor 10c is, for example, a computer such as a server or a desktop PC. As in the case of the information processor 10 in FIG. 1, the information processor 10c includes an input unit 11, a storage unit 12, a reception unit 13, a feature amount calculation unit 14, a digestibility estimation unit 15, a determination unit 16, a correction unit 17, an information presentation unit 18, and a table generation unit 19.

Next, description is given of a flow of processing executed by the information processor 10c to estimate the digestibility and present information to the user based on the estimation result.

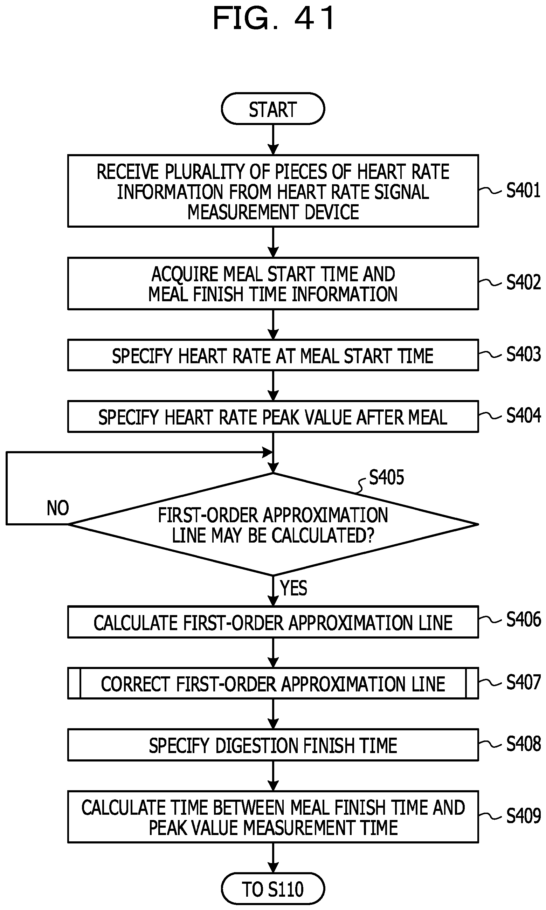

FIG. 41 is a flowchart (Part 1) illustrating an example of a digestibility estimation method executed by the information processor 10c in Example 3.

First, the reception unit 13 acquires a plurality of pieces of heart rate signal information from the heart rate signal measurement device 20 (S401). The processing of S401 is approximately the same as that of S101 in FIG. 4 in Example 1, and thus description thereof is omitted.

Then, the reception unit 13 acquires meal start time and meal finish time information from the information processor 10b (S402). To be more specific, the user carrying the information processor 10b inputs the meal start time and meal finish time information to the input unit 11. Then, the information processor 10b transmits the information inputted to the input unit 11 to a second receiver 13b in the information processor 10c. Thus, the information processor 10c may receive the meal start time and meal finish time information. Alternatively, the information processor 10c specifies the meal start time and the meal finish time by detecting the movement of the user with an acceleration sensor or an intracorporeal sound microphone worn by the user, and transmits the specified information to the reception unit 13 in the information processor 10c. Thus, the information processor 10c may receive the meal start time and meal finish time information.

The processing from S403 to S409 is approximately the same as the processing from S103 to S109 in FIG. 4 in Example 1, and thus description thereof is omitted.

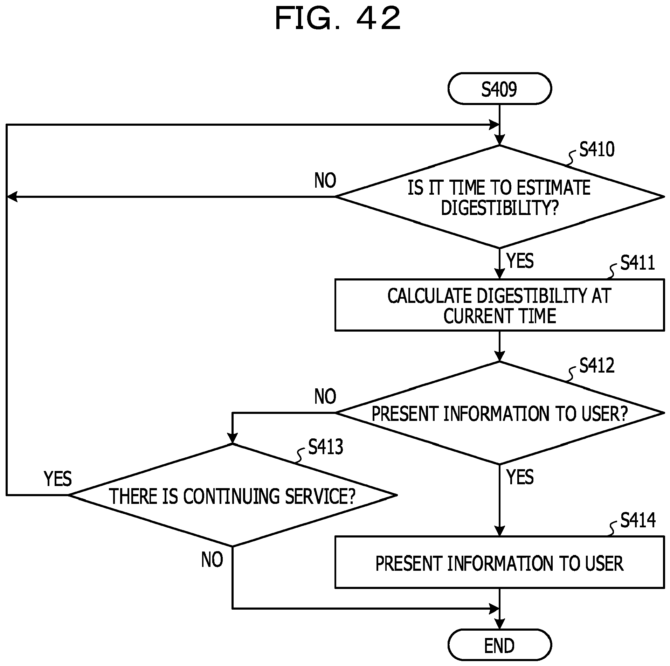

FIG. 42 is a flowchart (Part 2) illustrating an example of the digestibility estimation method executed by the information processor 10c in Example 3.

The processing from S410 to S412 is approximately the same as the processing from S110 to S112 in FIG. 27 in Example 1, and thus description thereof is omitted. When it is determined that information is not to be presented to the user in S412 (S412: No), the determination unit 16 determines whether or not there is a continuing service to the user (S413). In S412, the information processor 10c determines whether or not there is a continuing service to the user by determining, for example, whether or not a signal indicating the suspension of use of an application related to the service by the information processor 10b is received. When it is determined that there is a continuing service to the user (S413: Yes), the processing returns to S410 to execute again the processing of S410 and thereafter. When it is determined that there is no continuing service to the user (S413: No), a series of processing is terminated.

On the other hand, when it is determined that information is to be presented to the user in S412 (S412: Yes), the information presentation unit 18 transmits an instruction signal to instruct the presentation of information to the user to the information processor 10b (S414). Note that, when the information to be presented to the user includes information on a store such as a restaurant or a facility, and the like, the information processor 10c acquires the information to be presented to the user from an external device such as a server having a DB that stores related information through a network, for example. An instruction signal may also be transmitted together with the acquired information. Another method may also be adopted wherein the DB that stores related information is stored in the storage unit 12 in the information processor 10c and required information is read from the storage unit 12. Since the information processor 10c itself includes the DB, it is no longer required to communicate with the external device to acquire service information. After S414, a series of processing is terminated.

The information processor 10c installed in the place away from the user also executes the processing of generating a corrected value management table. To be more specific, the second receiver 13b in the information processor 10c receives heart rate-related data from a plurality of users through wireless communication, and the table generation unit 19 performs statistical processing on the received data to generate the corrected value management table 12e.

According to Example 3, the information processor 10c installed in the place away from the user, rather than the information processor 10b carried by the user, is in charge of the processing for estimating the digestibility and storage of various tables used to estimate the digestibility. Thus, load on a processor in the information processor carried by the user may be reduced, and the use of storage area in a memory included in the information processor may be reduced.

(EXAMPLE 4)

Next, Example 4 is described. In Examples 1 to 3, various tables used for processing of estimating the digestibility are stored in the information processor carried by the user. On the other hand, Example 4 is characterized in that various tables are stored in an external storage device rather than the information processor carried by the user.

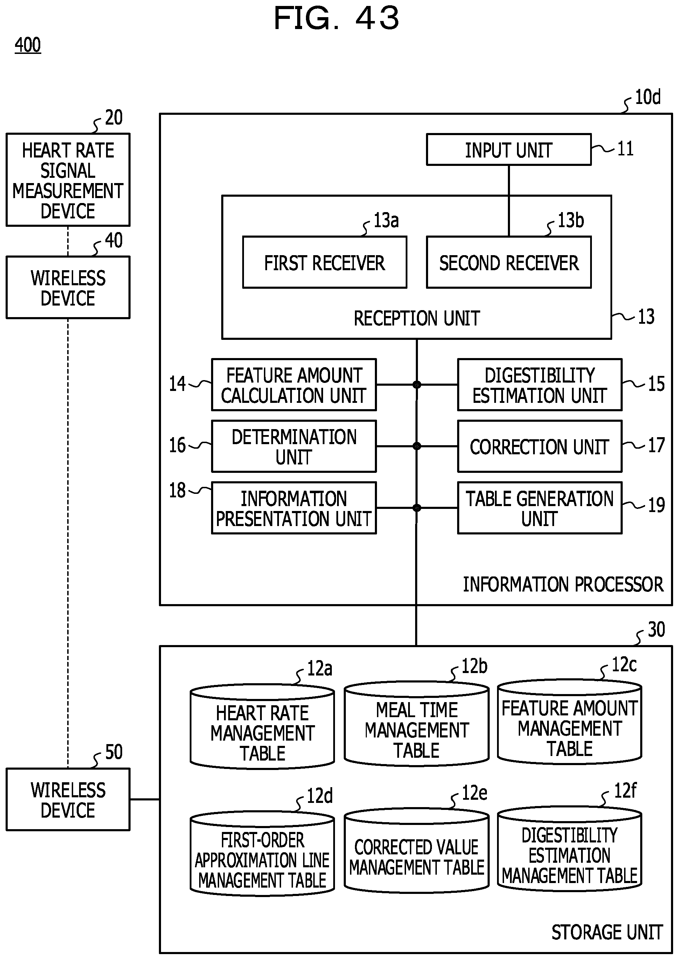

FIG. 43 is a diagram illustrating an example of an information processing system 400 in Example 4. The same functional blocks having the same functions as those of the functional blocks illustrated in FIG. 1 are denoted by the same reference numerals of those in FIG. 1, and description thereof is omitted. As illustrated in FIG. 43, the information processing system 400 includes an information processor 10d, a heart rate signal measurement device 20, a storage device 30, a wireless device 40, and a wireless device 50. The broken lines in FIG. 43 represent wireless communication.

As in the case of the information processor 10 in Example 1, the information processor 10d is a computer carried by the user. More specifically, the information processor 10d is a device that estimates a digestibility and provides services to the user based on the estimation result, as in the case of the information processor 10. The information processor 10d is, for example, a smartphone, a cell-phone, a tablet terminal, a laptop PC, a wearable computer, or the like. The information processor 10d may also be realized as a wearable computer including the heart rate signal measurement device 20. As in the case of the information processor 10 in FIG. 1, the information processor 10d includes an input unit 11, a reception unit 13, a feature amount calculation unit 14, a digestibility estimation unit 15, a determination unit 16, a correction unit 17, an information presentation unit 18, and a table generation unit 19. Note that, although not illustrated in FIG. 43, the information processor 10d includes a non-volatile memory used to store programs (such as a digestibility estimation program) and a cache memory that temporarily stores data to execute the programs.

The storage device 30 is used as a DB for various information used for processing to be executed by the information processor 10d, and is realized by a storage device or a server, for example. The information processor 10d may execute processing by reading appropriate data from the storage device 30 or storing data therein. The information processor 10d may use the storage device 30 as a cloud server by accumulating various information in the storage device 30.

The wireless device 40 is connected to the heart rate signal measurement device 20, while the wireless device 50 is connected to the storage device 30. The wireless devices 40 and 50 are realized by wireless interfaces, for example. The wireless devices 40 and 50 may be included in the heart rate signal measurement device 20 and the storage device 30, respectively.

The heart rate signal measurement device 20 transmits information of heart rates measured at specified intervals to the storage device 30 through wireless communication via the wireless devices 40 and 50 for each measurement cycle or at a predetermined batch timing. The heart rate information received by the storage device is sequentially accumulated in a heart rate management table 12a over time.

The processing of estimating a digestibility, which is executed by the information processor 10d, is started from S102 in FIG. 4. The information processor 10d executes the processing in S103 and thereafter while reading information required for the processing from various tables stored in the storage device 30 or referring to the various tables. The content of each processing is approximately the same as that in Example 1, and thus description thereof is omitted.

According to Example 4, the storage device that is the external device, rather than the information processor carried by the user, is in charge of the storage of various tables used to estimate the digestibility. Thus, the use of storage area in a memory included in the information processor carried by the user may be reduced.

Although the preferred examples of the present invention have been described in detail above, the present invention is not limited to these specific examples but various modifications and changes may be made thereto. For example, although the digestibility is estimated in this embodiment, the digestibility may also be replaced by a term such as a hunger level and a satiety level.

In the description of Example 1, for example, the first-order approximation line is calculated by using information of the plurality of heart rates measured between the peak value measurement time and the time after the setting time has elapsed since the peak value measurement time. However, when the current time is the time after the setting time has elapsed, the first-order approximation line may be calculated by using information of a plurality of heart rates measured between the peak value measurement time and the current time. According to this method, the longer the time after the setting time has elapsed, the more the amount of heart rate information used to calculate the first-order approximation line. Thus, the accuracy of the first-order approximation line is improved, and the digestion finish time may be more accurately specified.

Moreover, although the digestion finish time is specified by using the first-order approximation line in Examples 1 to 4, the digestion finish time may also be specified (not covered by the scope of the claims) by using a high-order curve such as a second-order curve. When the digestibility is estimated to be within a range of 50% to 100%, for example, sufficient heart rate information is accumulated, and thus the digestion finish time may be more accurately specified by using a high-order curve.

Moreover, when providing a service of recommending a favorable restaurant, for example, a different method may be used to display information on the restaurant to be recommended, depending on the calculated eating activity energy value. An example of this method is described with reference to the functional block diagram of the information processor 10 illustrated in FIG. 1.

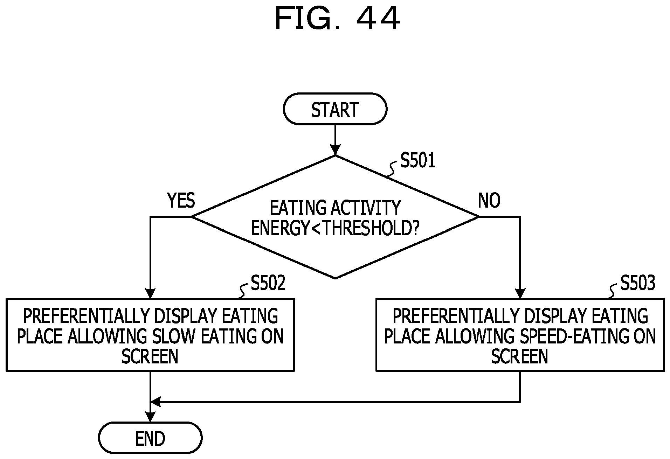

FIG. 44 is a diagram illustrating an example of a method (by itself not falling under the scope of the claims) for displaying information on a recommended restaurant. First, the determination unit 16 determines whether or not the eating activity energy is smaller than a predetermined threshold by comparing the eating activity energy with the threshold (S501). This determination processing is performed to determine whether or not the last meal is a light meal. Therefore, it is preferable that the threshold is set to a value smaller than an eating activity energy value when a meal supposed to be slowly eaten is eaten slowly.

When it is determined in S501 that the eating activity energy is smaller than the threshold (S501: Yes), the information presentation unit 18 preferentially displays an eating place, such as a restaurant, that allows slow eating, for example, on a screen (S502). More specifically, to display a plurality of eating place candidates, the information presentation unit 18 ranks eating places such that the eating place that allows slow eating ranks high, and displays the eating places in the ranking order on the screen.

On the other hand, when it is determined in S501 that the eating activity energy is not less than the threshold (S502: No), the information presentation unit 18 preferentially displays an eating place, such as a fast-food restaurant, that allows speed-eating on the screen (S503). More specifically, to display a plurality of eating place candidates, the information presentation unit 18 ranks eating places such that the eating place that allows speed-eating ranks high, and displays the eating places in the ranking order on the screen.

In this way, the information processor 10 may display the information of the recommended restaurants. With this method, services corresponding to the activity of the user may be provided to the user.

Note that a computer program that causes a computer to execute the portable terminal device and control method described above as well as a non-transitory computer-readable recording medium having the program recorded therein are included in the scope of the present disclosure. Here, the non-transitory computer-readable recording medium is, for example, a memory card such as an SD memory card. Note that the computer program is not limited to the one recorded in the recording medium, but may be transmitted through an electric communication line, a wireless or wired communication line, a network typified by the Internet, or the like.

Reference Signs List

| | |
|---|---|
| 10, 10a, 10b, 10c, 10d | INFORMATION PROCESSOR |
| 11 | INPUT UNIT |
| 12 | STORAGE UNIT |
| 12a | HEART RATE MANAGEMENT TABLE |
| 12b | MEAL TIME MANAGEMENT TABLE |

| 12c | FEATURE AMOUNT MANAGEMENT TABLE |
| 12d | FIRST-ORDER APPROXIMATION LINE MANAGEMENT TABLE |
| 12e | CORRECTED VALUE MANAGEMENT TABLE |
| 12f | DIGESTIBILITY ESTIMATION MANAGEMENT TABLE |
| 12g | FIRST-ORDER APPROXIMATION LINE MANAGEMENT TABLE |
| 12h | CORRECTED VALUE MANAGEMENT TABLE |
| 13 | RECEPTION UNIT |
| 13a | FIRST RECEIVER |
| 13b | SECOND RECEIVER |
| 14 | FEATURE AMOUNT CALCULATION UNIT |
| 15 | DIGESTIBILITY ESTIMATION UNIT |
| 16 | DETERMINATION UNIT |
| 17 | CORRECTION UNIT |
| 18 | INFORMATION PRESENTATION UNIT |
| 19 | TABLE GENERATION UNIT |
| 20 | HEART RATE SIGNAL MEASUREMENT DEVICE |
| 30 | STORAGE DEVICE |
| 40, 50 | WIRELESS DEVICE |
| 61 | CPU |
| 62 | ROM |
| 63 | RAM |
| 64 | STORAGE DEVICE |
| 65 | INPUT DEVICE |
| 66 | DISPLAY DEVICE |
| 67 | NETWORK INTERFACE |
| 68 | PORTABLE STORAGE MEDIUM DRIVE |
| 69 | PORTABLE STORAGE MEDIUM |
| 70 | BUS |
| 100, 200, 300, 400 | INFORMATION PROCESSING SYSTEM |

**Claims**

1. An information processor for estimating a digestibility of food consumed by a user, comprising:

a first receiver (13a) for receiving a plurality of pieces of heart rate information measured at specified time intervals from a heart rate signal measurement device carried by the user;
a second receiver (13b) for receiving information of a meal start time and a meal finish time of the user;
a line calculation unit (14) for calculating, based on a trend indicated by the plurality of pieces of heart rate information, in which the heart rate rises with time after meal and then drops, a first-order approximation line that approximates a change in the heart rate in a predetermined time slot of the trend after a time of measurement of a peak value of the heart rate by a line;
a correction unit (17) for correcting the first-order approximation line based on a trend indicated by the plurality of pieces of heart rate information in which the heart rate rises with time during meal and then drops;
a time specification unit (14) for specifying a digestion finish time indicating the time when digestion of the food is completed, based on the corrected first-order approximation line and the heart rate at the meal start time; and
a digestibility estimation unit (15) for estimating the digestibility at a current time, which is obtained by calculating an equation which is [(the current time - the meal finish time) / (a first time between the meal finish time and a time of measurement of the peak value indicating a maximum value of the heart rate after the meal + a second time between the time of measurement of the peak value and the digestion finish time)],
wherein the correction unit (17) is configured to execute processing of:

calculating (S201) an index value indicating eating activity energy from the trend in which the heart rate rises with time during meal and then drops,
extracting (S202) a corrected value corresponding to the index value calculated by the line calculation unit, from corrected value management information indicating a correspondence relationship between the index value and the corrected value, and
correcting (S203) the first-order approximation line with the corrected value.

2. The information processor according to claim 1, further comprising:

   a determination unit (16) for determining whether or not the digestibility exceeds a threshold; and
   an information presentation unit (18) for presenting service-related information to the user when the determination unit determines that the digestibility exceeds the threshold.

3. The information processor according to claim 1 or claim 2, wherein
   the index value is an area of a figure generated using a waveform the trend in which the heart rate rises with time during meal and then drops.

4. The information processor according to claim 1 or claim 2, wherein
   the index value is a rise rate when the heart rate rises with time during meal.

5. The information processor according to any one of claims 1 to 4, wherein
   the correction unit (17) is configured to correct the first-order approximation line by multiplying the slope of the first-order approximation line by the corrected value.

6. The information processor according to any one of claims 1 to 4, wherein
   the correction unit (17) is configured to correct the first-order approximation line by adding the corrected value to an intercept, of the first-order approximation line, on a coordinate axis representing the heart rate.

7. A digestibility estimation method executed by an information processor for estimating a digestibility of food consumed by a user carrying a heart rate signal measurement device, comprising:

   receiving (S101) a plurality of pieces of heart rate information measured at specified time intervals from the heart rate signal measurement device;
   receiving (S102) information of a meal start time and a meal finish time of the user from a device different from the heart rate signal measurement device;
   calculating (S106), based on a trend indicated by the plurality of pieces of heart rate information, in which the heart rate rises with time after meal and then drops, a first-order approximation line that approximates a change in the heart rate in a predetermined time slot of the trend after a time of measurement of a peak value of the heart rate by a line;
   correcting (S107) the first-order approximation line based on a trend indicated by the plurality of pieces of heart rate information in which the heart rate rises with time during meal and then drops;
   specifying (S108) a digestion finish time indicating the time when digestion of the food is completed, based on the corrected first-order approximation line and the heart rate at the meal start time; and
   estimating (S111) the digestibility at a current time, which is obtained by calculating an equation which is [(the current time - the meal finish time) / (a first time between the meal finish time and a time of measurement of the peak value indicating a maximum value of the heart rate after the meal + a second time between the time of measurement of the peak value and the digestion finish time)],
   wherein the correcting (S107) the first-order approximation line comprises:

   calculating (S201) an index value indicating eating activity energy from the trend in which the heart rate rises with time during meal and then drops,
   extracting (S202) a corrected value corresponding to the index value calculated by the line calculation unit, from corrected value management information indicating a correspondence relationship between the index value and the corrected value, and
   correcting (S203) the first-order approximation line with the corrected value.

8. An information processing system for estimating a digestibility of food consumed by a user, comprising:

   a heart rate signal measurement device; and
   the information processor according to any of claims 1 to 6, the information processor communicably connected to the heart rate signal measurement device.

9. A digestibility estimation program for causing an information processor for estimating a digestibility of a user carrying a heart rate signal measurement device and a portable terminal device to execute processing of:

receiving (S101) a plurality of pieces of heart rate information measured at specified time intervals from the heart rate signal measurement device;

receiving (S102) information of a meal start time and a meal finish time of the user from a device different from the heart rate signal measurement device;

calculating (S106), based on a trend indicated by the plurality of pieces of heart rate information, in which the heart rate rises with time after meal and then drops, a first-order approximation line that approximates a change in the heart rate in a predetermined time slot of the trend after a time of measurement of a peak value of the heart rate by a line;

correcting (S107) the first-order approximation line based on a trend indicated by the plurality of pieces of heart rate information in which the heart rate rises with time during meal and then drops;

specifying (S108) a digestion finish time indicating the time when digestion of the food is completed, based on the corrected first-order approximation line and the heart rate at the meal start time; and

estimating (S111) the digestibility at a current time, which is obtained by calculating an equation which is [(the current time - the meal finish time) / (a first time between the meal finish time and a time of measurement of the peak value indicating a maximum value of the heart rate after the meal + a second time between the time of measurement of the peak value and the digestion finish time)],

wherein the correcting (S107) the first-order approximation line comprises:

calculating (S201) an index value indicating eating activity energy from the trend in which the heart rate rises with time during meal and then drops,

extracting (S202) a corrected value corresponding to the index value calculated by the line calculation unit, from corrected value management information indicating a correspondence relationship between the index value and the corrected value, and

correcting (S203) the first-order approximation line with the corrected value.

## Patentansprüche

1. Informationsprozessor zur Schätzung einer Verdaulichkeit von Nahrung, die von einem Benutzer verzehrt wird, umfassend:

einen ersten Empfänger (13a) zum Empfangen einer Vielzahl von Herzfrequenzinformationen, die in spezifizierten Zeitintervallen von einer von dem Benutzer mitgeführten Herzfrequenzsignal-Messvorrichtung gemessen werden;

einen zweiten Empfänger (13b) zum Empfangen von Informationen über eine Mahlzeit-Startzeit und eine Mahlzeit-Endzeit des Benutzers;

eine Linienberechnungseinheit (14) zum Berechnen, basierend auf einem durch die Vielzahl von Herzfrequenzinformationen angezeigten Trend, bei dem die Herzfrequenz mit der Zeit nach dem Essen ansteigt und dann abfällt, einer Näherungslinie erster Ordnung, die eine Änderung der Herzfrequenz in einem vorbestimmten Zeitschlitz des Trends nach einer Zeit einer Messung eines Spitzenwertes der Herzfrequenz durch eine Linie approximiert;

eine Korrektureinheit (17) zum Korrigieren der Näherungslinie erster Ordnung basierend auf einem durch die Vielzahl von Herzfrequenzinformationen angezeigten Trend, bei dem Herzfrequenz während der Mahlzeit mit der Zeit ansteigt und dann abfällt;

eine Zeitspezifikationseinheit (14) zum Spezifizieren einer Verdauungs-Endzeit, die die Zeit anzeigt, zu der die Verdauung der Nahrung abgeschlossen ist, basierend auf der korrigierten Näherungslinie erster Ordnung und der Herzfrequenz zur Mahlzeit-Startzeit; und

eine Verdaulichkeits-Schätzeinheit (15) zum Schätzen der Verdaulichkeit zu einer aktuellen Zeit, die durch Berechnen einer Gleichung erhalten wird, die lautet [(aktuelle Zeit - Mahlzeit-Endzeit)/(eine erste Zeit zwischen der Mahlzeit-Endzeit und einer Zeit einer Messung des Spitzenwertes, der einen Maximalwert der Herzfrequenz nach der Mahlzeit anzeigt + eine zweite Zeit zwischen der Zeit der Messung des Spitzenwertes und der Verdauungs-Endzeit)],

wobei die Korrektureinheit (17) dazu ausgelegt ist, eine Verarbeitung auszuführen von:

Berechnen (S201) eines Indexwertes, der die Essaktivitätsenergie anzeigt, aus dem Trend, bei dem die Herzfrequenz während einer Mahlzeit mit der Zeit ansteigt und dann abfällt,

Extrahieren (S202) eines korrigierten Wertes, der dem durch die Zeilenberechnungseinheit berechneten Indexwert entspricht, aus Korrekturwert-Verwaltungsinformationen, die eine Korrespondenzbeziehung zwischen dem Indexwert und dem korrigierten Wert anzeigen, und
Korrigieren (S203) der Näherungslinie erster Ordnung mit dem korrigierten Wert.

2. Informationsprozessor nach Anspruch 1, ferner umfassend:

eine Bestimmungseinheit (16) zum Bestimmen, ob die Verdaulichkeit einen Schwellenwert überschreitet oder nicht; und
eine Informationspräsentationseinheit (18) zum Präsentieren von dienstbezogenen Informationen an den Benutzer, wenn die Bestimmungseinheit bestimmt, dass die Verdaulichkeit den Schwellenwert überschreitet.

3. Informationsprozessor nach Anspruch 1 oder Anspruch 2, wobei
der Indexwert ist ein Bereich einer Figur ist, die unter Verwendung einer Kurvenform erzeugt wird, die den Trend zeigt, bei dem die Herzfrequenz während der Mahlzeit mit der Zeit ansteigt und dann abfällt.

4. Informationsprozessor nach Anspruch 1 oder Anspruch 2, wobei
der Indexwert eine Anstiegsrate ist, wenn die Herzfrequenz während Mahlzeit mit der Zeit ansteigt.

5. Informationsprozessor nach einem der Ansprüche 1 bis 4, wobei
die Korrektureinheit (17) dazu ausgelegt ist, die Näherungslinie erster Ordnung durch Multiplizieren der Steigung der Näherungslinie erster Ordnung mit dem korrigierten Wert zu korrigieren.

6. Informationsprozessor nach einem der Ansprüche 1 bis 4, wobei
die Korrektureinheit (17) dazu ausgelegt ist, die Näherungslinie erster Ordnung durch Addieren des korrigierten Wertes zu einem Schnittpunkt, der Näherungslinie erster Ordnung, auf einer die Herzfrequenz repräsentierenden Koordinatenachse zu korrigieren.

7. Verfahren zur Schätzung der Verdaulichkeit, ausgeführt von einem Informationsprozessor zur Schätzung einer Verdaulichkeit von Nahrung, die von einem Benutzer verzehrt wird, der eine Herzfrequenzsignal-Messvorrichtung trägt, umfassend:

Empfangen (S101) einer Vielzahl von Herzfrequenzinformationen, die in spezifizierten Zeitintervallen von der Herzfrequenzsignal-Messvorrichtung gemessen werden;
Empfangen (S102) von Informationen über eine Mahlzeit-Startzeit und eine Mahlzeit-Endzeit des Benutzers von einer Vorrichtung, die sich von der Herzfrequenzsignal-Messvorrichtung unterscheidet;
Berechnen (S106), basierend auf einem durch die Vielzahl von Herzfrequenzinformationen angezeigten Trend, bei dem die Herzfrequenz mit der Zeit nach dem Essen ansteigt und dann abfällt, einer Näherungslinie erster Ordnung, die eine Änderung der Herzfrequenz in einem vorbestimmten Zeitschlitz des Trends nach einer Zeit einer Messung eines Spitzenwertes der Herzfrequenz durch eine Linie approximiert;
Korrigieren (S107) der Näherungslinie erster Ordnung basierend auf einem durch die Vielzahl von Herzfrequenzinformationen angezeigten Trend, bei dem Herzfrequenz während der Mahlzeit mit der Zeit ansteigt und dann abfällt;
Spezifizieren (S108) einer Verdauungs-Endzeit, die die Zeit anzeigt, zu der die Verdauung der Nahrung abgeschlossen ist, basierend auf der korrigierten Näherungslinie erster Ordnung und der Herzfrequenz zur Mahlzeit-Startzeit; und
Schätzen (S111) der Verdaulichkeit zu einer aktuellen Zeit, die durch Berechnen einer Gleichung erhalten wird, die lautet [(aktuelle Zeit - Mahlzeit-Endzeit)/(eine erste Zeit zwischen der Mahlzeit-Endzeit und einer Zeit einer Messung des Spitzenwertes, der einen Maximalwert der Herzfrequenz nach der Mahlzeit anzeigt + eine zweite Zeit zwischen der Zeit der Messung des Spitzenwertes und der Verdauungs-Endzeit)],
wobei das Korrigieren (S107) der Näherungslinie erster Ordnung umfasst:

Berechnen (S201) eines Indexwertes, der eine Essaktivitätsenergie anzeigt, aus dem Trend, bei dem die Herzfrequenz während der Mahlzeit mit der Zeit ansteigt und dann abfällt,
Extrahieren (S202) eines korrigierten Wertes, der dem durch die Zeilenberechnungseinheit berechneten Indexwert entspricht, aus Korrekturwert-Verwaltungsinformationen, die eine Korrespondenzbeziehung zwischen dem Indexwert und dem korrigierten Wert anzeigen, und
Korrigieren (S203) der Näherungslinie erster Ordnung mit dem korrigierten Wert.

8. Informationsverarbeitungssystem zur Schätzung einer Verdaulichkeit von Nahrung, die von einem Benutzer verzehrt wird, umfassend:

eine Herzfrequenzsignal-Messvorrichtung; und
einen Informationsprozessor nach einem der Ansprüche 1 bis 6, wobei der Informationsprozessor kommunikativ mit der Herzfrequenzsignal-Messvorrichtung verbunden ist.

**9.** Verdaulichkeits-Schätzprogramm zum Bewirkung, dass ein Informationsprozessor zur Schätzung der Verdaulichkeit, für einen Benutzer, der eine Herzfrequenzsignal-Messvorrichtung und ein tragbares Endgerät trägt, eine Verarbeitung auszuführt von:

Empfangen (S101) einer Vielzahl von Herzfrequenzinformationen, die in spezifizierten Zeitintervallen von der Herzfrequenzsignal-Messvorrichtung gemessen werden;
Empfangen (S102) von Informationen über eine Mahlzeit-Startzeit und eine Mahlzeit-Endzeit des Benutzers von einer anderen Vorrichtung als der Herzfrequenzsignal-Messvorrichtung;
Berechnen (S106), basierend auf einem durch die Vielzahl von Herzfrequenzinformationen angezeigten Trend, bei dem Herzfrequenz mit der Zeit nach dem Essen ansteigt und dann abfällt, einer Näherungslinie erster Ordnung, die eine Änderung der Herzfrequenz in einem vorbestimmten Zeitschlitz des Trends nach einer Zeit der Messung eines Spitzenwertes der Herzfrequenz durch eine Linie approximiert;
Korrigieren (S107) der Näherungslinie erster Ordnung basierend auf einem durch die Vielzahl von Herzfrequenzinformationen angezeigten Trend, bei dem Herzfrequenz während der Mahlzeit mit der Zeit ansteigt und dann abfällt;
Spezifizieren (S108) einer Verdauungs-Endzeit, die die Zeit anzeigt, zu der Verdauung der Nahrung abgeschlossen ist, basierend auf der korrigierten Näherungslinie erster Ordnung und der Herzfrequenz zur Mahlzeit-Startzeit; und
Schätzen (S111) der Verdaulichkeit zu einer aktuellen Zeit, die durch Berechnen einer Gleichung erhalten wird, die lautet [(aktuelle Zeit - Mahlzeit-Endzeit)/(eine erste Zeit zwischen der Mahlzeit-Endzeit und einer Zeit einer Messung des Spitzenwertes, der einen Maximalwert der Herzfrequenz nach der Mahlzeit anzeigt + eine zweite Zeit zwischen der Zeit der Messung des Spitzenwertes und der Verdauungs-Endzeit)],
wobei das Korrigieren (S107) der Näherungslinie erster Ordnung umfasst:

Berechnen (S201) eines Indexwertes, der eine Essaktivitätsenergie anzeigt, aus dem Trend, bei dem die Herzfrequenz während der Mahlzeit mit der Zeit ansteigt und dann abfällt,
Extrahieren (S202) eines korrigierten Wertes, der dem durch die Zeilenberechnungseinheit berechneten Indexwert entspricht, aus Korrekturwert-Verwaltungsinformationen, die eine Korrespondenzbeziehung zwischen dem Indexwert und dem korrigierten Wert anzeigen, und
Korrigieren (S203) der Näherungslinie erster Ordnung mit dem korrigierten Wert.

## Revendications

**1.** Processeur d'informations pour estimer une digestibilité d'aliments consommés par un utilisateur, comprenant :

un premier récepteur (13a) pour recevoir une pluralité d'informations de fréquence cardiaque mesurées à des intervalles de temps spécifiés à partir d'un dispositif de mesure de signal de fréquence cardiaque porté par l'utilisateur ;
un deuxième récepteur (13b) pour recevoir des informations d'une heure de début de repas et d'une heure de fin de repas de l'utilisateur ;
une unité de calcul de ligne (14) pour calculer, sur la base d'une tendance indiquée par la pluralité d'informations de fréquence cardiaque, dans laquelle la fréquence cardiaque augmente au fil du temps après le repas, puis diminue, une ligne d'approximation de premier ordre qui se rapproche d'une variation de la fréquence cardiaque dans un intervalle de temps prédéterminé de la tendance après un temps de mesure d'une valeur de crête de la fréquence cardiaque par une ligne ;
une unité de correction (17) pour corriger la ligne d'approximation du premier ordre sur la base d'une tendance indiquée par la pluralité d'informations de fréquence cardiaque dans laquelle la fréquence cardiaque augmente au fil du temps pendant le repas puis diminue ;
une unité de spécification de temps (14) pour spécifier une heure de fin de digestion indiquant l'heure à laquelle la digestion de l'aliment est terminée, sur la base de la ligne d'approximation de premier ordre corrigée et de la fréquence cardiaque à l'heure de début du repas ; et

une unité d'estimation de digestibilité (15) pour estimer la digestibilité à une heure actuelle, qui est obtenue en calculant une équation qui est [(l'heure actuelle - l'heure de fin de repas)/(une première fois entre l'heure de fin de repas et une heure de mesure de la valeur de crête indiquant une valeur maximale de la fréquence cardiaque après le repas + une deuxième fois entre l'heure de mesure de la valeur de crête et l'heure de fin de digestion)], dans lequel l'unité de correction (17) est configurée pour exécuter le traitement consistant à:

calculer (S201) une valeur d'index indiquant l'énergie d'activité de consommation à partir de la tendance selon laquelle la fréquence cardiaque augmente au fil du temps pendant le repas puis diminue, extraire (S202) une valeur corrigée correspondant à la valeur d'indice calculée par l'unité de calcul de ligne, à partir d'informations de gestion de valeur corrigée indiquant une relation de correspondance entre la valeur d'indice et la valeur corrigée, et corriger (S203) la ligne d'approximation du premier ordre avec la valeur corrigée.

2. Processeur d'informations selon la revendication 1, comprenant en outre :

une unité de détermination (16) pour déterminer si oui ou non la digestibilité dépasse un seuil; et une unité de présentation d'informations (18) pour présenter des informations relatives au service à l'utilisateur lorsque l'unité de détermination détermine que la digestibilité dépasse le seuil.

3. Processeur d'informations selon la revendication 1 ou la revendication 2, dans lequel la valeur de l'indice est une zone d'un chiffre généré à l'aide d'une forme d'onde la tendance dans laquelle la fréquence cardiaque augmente au fil du temps pendant le repas, puis diminue.

4. Processeur d'informations selon la revendication 1 ou la revendication 2, dans lequel la valeur de l'indice est une fréquence d'augmentation lorsque la fréquence cardiaque augmente au fil du temps pendant le repas.

5. Processeur d'informations selon une quelconque des revendications 1 à 4, dans lequel l'unité de correction (17) est configurée pour corriger la ligne d'approximation du premier ordre en multipliant la pente de la ligne d'approximation du premier ordre par la valeur corrigée.

6. Processeur d'informations selon une quelconque des revendications 1 à 4, dans lequel l'unité de correction (17) est configurée pour corriger la ligne d'approximation de premier ordre en ajoutant la valeur corrigée à une intersection, de la ligne d'approximation de premier ordre, sur un axe de coordonnées représentant la fréquence cardiaque.

7. Procédé d'estimation de la digestibilité exécuté par un processeur d'informations pour estimer la digestibilité d'un aliment consommé par un utilisateur portant un dispositif de mesure de signal de fréquence cardiaque, comprenant de:

recevoir (S101) une pluralité d'informations de fréquence cardiaque mesurées à des intervalles de temps spécifiés à partir du dispositif de mesure de signal de fréquence cardiaque ; recevoir (S102) des informations d'une heure de début de repas et d'une heure de fin de repas de l'utilisateur d'un appareil différent de l'appareil de mesure du signal de fréquence cardiaque ; calculer (S106), sur la base d'une tendance indiquée par la pluralité d'éléments d'informations relatifs à la fréquence cardiaque, dans lesquelles la fréquence cardiaque augmente au fil du temps après le repas, puis diminue ensuite, une ligne d'approximation de premier ordre qui se rapproche d'une variation de la fréquence cardiaque dans une plage horaire prédéterminée de la tendance après un temps de mesure d'une valeur de crête de la fréquence cardiaque par une ligne; corriger (S107) la ligne d'approximation de premier ordre sur la base d'une tendance indiquée par la pluralité d'informations de fréquence cardiaque dans laquelle la fréquence cardiaque au fil du temps pendant le repas puis diminue ; spécifier (S108) une heure de fin de digestion indiquant l'heure à laquelle la digestion de l'aliment est terminée, sur la base de la ligne d'approximation de premier ordre corrigée et de la fréquence cardiaque à l'heure de début de repas ; et estimer (S111) la digestibilité à une heure actuelle, qui est obtenue en calculant une équation qui est [(l'heure actuelle - l'heure de fin de repas) (une première fois entre l'heure de fin de repas et un moment de mesure de la valeur de crête indiquant une valeur maximale de la fréquence cardiaque après le repas + une deuxième fois entre le moment de la mesure de la valeur de crête et la fin de l'heure de digestion)], dans lequel la correction (S107) de la ligne d'approximation du premier ordre comprend de:

calculer (S201) une valeur d'indice indiquant l'énergie d'activité alimentaire à partir de la tendance dans laquelle la fréquence cardiaque augmente au fil du temps pendant le repas puis diminue,

extraire (S202) une valeur corrigée correspondant à la valeur d'indice calculée par l'unité de calcul de ligne, à partir d'informations de gestion de valeur corrigée indiquant une relation de correspondance entre la valeur d'indice et la valeur corrigée, et

corriger (S203) la ligne d'approximation du premier ordre avec la valeur corrigée.

8. Système de traitement de l'information pour estimer la digestibilité des aliments consommés par un utilisateur, comprenant :

un dispositif de mesure de signal de fréquence cardiaque ; et

le processeur d'informations selon une quelconque des revendications 1 à 6, les informations de processeur étant connectées de manière communicante au dispositif de mesure du signal de fréquence cardiaque.

9. Programme d'estimation de la digestibilité pour amener un processeur d'informations à estimer la digestibilité d'un utilisateur portant un dispositif de mesure de signal de fréquence cardiaque et un dispositif de terminal portable à exécuter le traitement consistant à :

recevoir (S101) une pluralité d'informations de fréquence cardiaque mesurées à des intervalles de temps spécifiés à partir du dispositif de mesure de signal de fréquence cardiaque ;

recevoir (S102) des informations d'une heure de début de repas et d'une heure de fin de repas de l'utilisateur d'un appareil différent de l'appareil de mesure du signal de fréquence cardiaque ;

calculer (S106), sur la base d'une tendance indiquée par la pluralité d'éléments informations relatifs à la fréquence cardiaque, dans lequel la fréquence cardiaque augmente au fil du temps après le repas, puis diminue, une ligne d'approximation de premier ordre qui se rapproche d'un changement de la fréquence cardiaque dans une plage horaire prédéterminée d'une valeur de crête de la fréquence cardiaque par une ligne;

corriger (S107) la ligne d'approximation du premier ordre sur la base d'une tendance indiquée par la pluralité d'informations de fréquence cardiaque dans lequel la fréquence cardiaque augmente au fil du temps pendant le repas puis diminue ;

spécifier (S108) une heure de fin de digestion indiquant l'heure à laquelle la digestion de l'aliment est terminée, sur la base de la ligne d'approximation de premier ordre corrigée et de la fréquence cardiaque à l'heure de début de repas ; et

estimer (S111) la digestibilité à une heure actuelle, qui est obtenue en calculant une équation qui est [(l'heure actuelle - l'heure de fin de repas) / (une première fois entre l'heure de fin de repas et un moment de mesure de la valeur de crête indiquant une valeur maximale de la fréquence cardiaque après le repas + une deuxième fois entre l'heure de mesure de la valeur de crête et l'heure de fin de digestion)],

dans lequel la correction (S107) de la ligne d'approximation du premier ordre comprend de :

calculer (S201) une valeur d'indice indiquant l'énergie d'activité alimentaire à partir de la tendance dans laquelle la fréquence cardiaque augmente au fil du temps pendant le repas puis diminue,

extraire (S202) une valeur corrigée correspondant à la valeur d'indice calculée par l'unité de calcul de ligne, à partir d'informations de gestion de valeur corrigée indiquant une relation de correspondance entre la valeur d'indice et la valeur corrigée, et

corriger (S203) la ligne d'approximation du premier ordre avec la valeur corrigée.

# FIG. 1

100

```
┌─────────────────────┐
│  HEART RATE SIGNAL  │
│     MEASUREMENT     │~20
│       DEVICE        │
└─────────────────────┘
```

10

┌──────────────────────────────────────┐
│                  INPUT UNIT  ~11      │
│                                       │
│  ┌─13a            ┌─13b              │
│  FIRST RECEIVER   SECOND RECEIVER    │
│                                       │
│         RECEPTION UNIT      ~13       │
│                                       │
│  14~ FEATURE AMOUNT      DIGESTIBILITY ~15  │
│      CALCULATION UNIT    ESTIMATION UNIT    │
│                                       │
│  16~ DETERMINATION       CORRECTION UNIT ~17 │
│         UNIT                          │
│                                       │
│  18~ INFORMATION         TABLE GENERATION ~19 │
│      PRESENTATION UNIT       UNIT     │
│                                       │
│                              12       │
│  ┌─12a         ┌─12b         ┌─12c   │
│  HEART RATE    MEAL TIME     FEATURE AMOUNT │
│  MANAGEMENT    MANAGEMENT    MANAGEMENT TABLE │
│  TABLE         TABLE                  │
│                                       │
│  ┌─12d         ┌─12e         ┌─12f   │
│  FIRST-ORDER   CORRECTED VALUE  DIGESTIBILITY │
│  APPROXIMATION MANAGEMENT TABLE ESTIMATION │
│  LINE                        MANAGEMENT TABLE │
│  MANAGEMENT TABLE                     │
│                                       │
│                         STORAGE UNIT  │
│                                       │
│                  INFORMATION PROCESSOR │
└──────────────────────────────────────┘

# FIG. 2

10

INFORMATION PROCESSOR

| 61 | 65 | | 66 |
|---|---|---|---|
| CPU | INPUT DEVICE | | DISPLAY DEVICE |

70

| 62 | 63 | 64 | 67 | 68 |
|---|---|---|---|---|
| ROM | RAM | STORAGE DEVICE | NETWORK INTERFACE | PORTABLE STORAGE MEDIUM DRIVE |

69

PORTABLE STORAGE MEDIUM

# FIG. 3

# FIG. 4

```
                    ┌─────────┐
                    │  START  │
                    └─────────┘
                         │
                         ▼
    ┌─────────────────────────────────────────┐
    │ RECEIVE PLURALITY OF PIECES OF HEART RATE │ ~ S101
    │ INFORMATION FROM HEART RATE SIGNAL        │
    │ MEASUREMENT DEVICE                        │
    └─────────────────────────────────────────┘
                         │
                         ▼
    ┌─────────────────────────────────────────┐
    │      ACQUIRE MEAL START TIME AND          │ ~ S102
    │      MEAL FINISH TIME INFORMATION         │
    └─────────────────────────────────────────┘
                         │
                         ▼
    ┌─────────────────────────────────────────┐
    │   SPECIFY HEART RATE AT MEAL START TIME   │ ~ S103
    └─────────────────────────────────────────┘
                         │
                         ▼
    ┌─────────────────────────────────────────┐
    │   SPECIFY HEART RATE PEAK VALUE AFTER MEAL │ ~ S104
    └─────────────────────────────────────────┘
                         │
                         ▼
                    ╱─────────────╲          S105
              NO  ╱  FIRST-ORDER    ╲
           ┌─────  APPROXIMATION      ─────
           │     ╲  LINE MAY BE      ╱
           │       ╲ CALCULATED?   ╱
           │         ╲───────────╱
           │              │ YES
           │              ▼
           │  ┌─────────────────────────────────────────┐
           │  │  CALCULATE FIRST-ORDER APPROXIMATION LINE │ ~ S106
           │  └─────────────────────────────────────────┘
           │              │
           │              ▼
           │  ┌─────────────────────────────────────────┐
           │  │  CORRECT FIRST-ORDER APPROXIMATION LINE   │ ~ S107
           │  └─────────────────────────────────────────┘
                          │
                          ▼
              ┌─────────────────────────────────────────┐
              │      SPECIFY DIGESTION FINISH TIME        │ ~ S108
              └─────────────────────────────────────────┘
                          │
                          ▼
              ┌─────────────────────────────────────────┐
              │ CALCULATE TIME BETWEEN MEAL FINISH TIME AND│ ~ S109
              │ PEAK VALUE MEASUREMENT TIME               │
              └─────────────────────────────────────────┘
                          │
                          ▼
                     ┌─────────┐
                     │ TO S110 │
                     └─────────┘
```

# FIG. 5

12a

| TIME OF MEASUREMENT [hh:mm] | HEART RATE [bpm] |
|---|---|
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |
|  |  |

# FIG. 6

12a

| TIME OF MEASUREMENT [hh:mm] | HEART RATE [bpm] |
|---|---|
| 12:00 | 80 |
| 12:01 | 80 |
| 12:02 | 80 |
| 12:03 | 90 |
| 12:04 | 100 |
| 12:05 | 120 |
| 12:06 | 100 |
| 12:07 | 80 |
| 12:08 | 82 |
| 12:09 | 84 |
| 12:10 | 86 |
| 12:11 | 88 |
| 12:12 | 90 |
| 12:13 | 92 |
| ... | ... |
| 12:20 | 90 |
| ... | ... |
| 12:25 | 100 |
| ... | ... |
| 12:35 | 99 |
| ... | ... |
| 12:45 | 98 |
| ... | ... |

# FIG. 7

| MEAL ID | MEAL START TIME [hh:mm] | MEAL FINISH TIME [hh:mm] |
|---------|------------------------|-------------------------|
|         |                        |                         |
|         |                        |                         |
|         |                        |                         |

12b

# FIG. 8

12b

| MEAL ID | MEAL START TIME [hh:mm] | MEAL FINISH TIME [hh:mm] |
|---------|-------------------------|--------------------------|
| 1 | 12:02 | 12:07 |
| 2 | | |
| ... | | |

# FIG. 9

| MEAL ID | FEATURE AMOUNT 1 | FEATURE AMOUNT 2 | FEATURE AMOUNT 3 | FEATURE AMOUNT 4 | FEATURE AMOUNT 5 | FEATURE AMOUNT 6 |
|---|---|---|---|---|---|---|
| | HEART RATE AT MEAL START TIME [bpm] | PEAK VALUE [bpm] | FIRST-ORDER APPROXIMATION LINE ax+b | DIGESTION FINISH TIME [hh:mm] | TIME BETWEEN MEAL FINISH TIME AND PEAK VALUE MEASUREMENT TIME [min] | TIME BETWEEN PEAK VALUE MEASUREMENT TIME AND DIGESTION FINISH TIME [min] |
| 1 | | | | | | |
| 2 | | | | | | |
| ... | | | | | | |

12C

# FIG. 10

| MEAL ID | FEATURE AMOUNT 1 | FEATURE AMOUNT 2 | FEATURE AMOUNT 3 | FEATURE AMOUNT 4 | FEATURE AMOUNT 5 | FEATURE AMOUNT 6 |
|---|---|---|---|---|---|---|
| | HEART RATE AT MEAL START TIME [bpm] | PEAK VALUE [bpm] | FIRST-ORDER APPROXIMATION LINE ax+b | DIGESTION FINISH TIME [hh:mm] | TIME BETWEEN MEAL FINISH TIME AND PEAK VALUE MEASUREMENT TIME [min] | TIME BETWEEN PEAK VALUE MEASUREMENT TIME AND DIGESTION FINISH TIME [min] |
| 1 | 80 | | | | | |
| 2 | | | | | | |
| ... | | | | | | |

12C

EP 3 488 773 B1

# FIG. 11

| MEAL ID | FEATURE AMOUNT 1<br>HEART RATE AT MEAL START TIME [bpm] | FEATURE AMOUNT 2<br>PEAK VALUE [bpm] | FEATURE AMOUNT 3<br>FIRST-ORDER APPROXIMATION LINE ax+b | FEATURE AMOUNT 4<br>DIGESTION FINISH TIME [hh:mm] | FEATURE AMOUNT 5<br>TIME BETWEEN MEAL FINISH TIME AND PEAK VALUE MEASUREMENT TIME [min] | FEATURE AMOUNT 6<br>TIME BETWEEN PEAK VALUE MEASUREMENT TIME AND DIGESTION FINISH TIME [min] |
|---|---|---|---|---|---|---|
| 1 | 80 | 100 | | | | |
| 2 | | | | | | |
| ... | | | | | | |

12C

# FIG. 12

## FIG. 13

EP 3 488 773 B1

12C

| MEAL ID | FEATURE AMOUNT 1 | FEATURE AMOUNT 2 | FEATURE AMOUNT 3 | FEATURE AMOUNT 4 | FEATURE AMOUNT 5 | FEATURE AMOUNT 6 |
|---|---|---|---|---|---|---|
| | HEART RATE AT MEAL START TIME [bpm] | PEAK VALUE [bpm] | FIRST-ORDER APPROXIMATION LINE ax+b | DIGESTION FINISH TIME [hh:mm] | TIME BETWEEN MEAL FINISH TIME AND PEAK VALUE MEASUREMENT TIME [min] | TIME BETWEEN PEAK VALUE MEASUREMENT TIME AND DIGESTION FINISH TIME [min] |
| 1 | 80 | 100 | -0.1×+100 | | | |
| 2 | | | | | | |
| ... | | | | | | |

# FIG. 14

# FIG. 15

# FIG. 16

```
        ┌──────────────┐
        │     S106     │
        └──────┬───────┘
               ↓
┌────────────────────────────────────────┐
│    CALCULATE EATING ACTIVITY ENERGY     │── S201 ┐
└────────────────────┬───────────────────┘        │
                     ↓                             │
┌────────────────────────────────────────┐        │
│ EXTRACT CORRECTED VALUE CORRESPONDING TO│        │
│ CALCULATED EATING ACTIVITY ENERGY BY    │── S202 ├─ S107
│ REFERRING TO CORRECTED VALUE            │        │
│ MANAGEMENT TABLE                        │        │
└────────────────────┬───────────────────┘        │
                     ↓                             │
┌────────────────────────────────────────┐        │
│ CORRECT SLOPE OF FIRST-ORDER            │── S203 ┘
│ APPROXIMATION LINE WITH EXTRACTED       │
│ CORRECTED VALUE                         │
└────────────────────┬───────────────────┘
                     ↓
        ┌──────────────┐
        │   TO S108    │
        └──────────────┘
```

# FIG. 17

# FIG. 18

# FIG. 19

# FIG. 20

| MEAL ID | EATING ACTIVITY ENERGY | CORRECTED VALUE $\alpha$ | CORRECTED FIRST-ORDER APPROXIMATION LINE cx+b |
|---------|------------------------|--------------------------|-----------------------------------------------|
| 1       |                        |                          |                                               |
| 2       |                        |                          |                                               |
| ...     |                        |                          |                                               |

12d

# FIG. 21

12d

| MEAL ID | EATING ACTIVITY ENERGY | CORRECTED VALUE $\alpha$ | CORRECTED FIRST-ORDER APPROXIMATION LINE $cx+b$ |
|---------|------------------------|--------------------------|-------------------------------------------------|
| 1 | 140 | | |
| 2 | | | |
| ... | | | |

# FIG. 22

12e

| EATING ACTIVITY ENERGY | CORRECTED VALUE $\alpha$ |
|:---:|:---:|
| ... | ... |
| 131 TO 150 | 0.8 |
| 111 TO 130 | 1.0 |
| 90 TO 110 | 1.2 |
| ... | ... |

# FIG. 23

12d

| MEAL ID | EATING ACTIVITY ENERGY | CORRECTED VALUE $\alpha$ | CORRECTED FIRST-ORDER APPROXIMATION LINE $cx+b$ |
|---|---|---|---|
| 1 | 140 | 0.8 | |
| 2 | | | |
| ... | | | |

# FIG. 24

12d

| MEAL ID | EATING ACTIVITY ENERGY | CORRECTED VALUE $\alpha$ | CORRECTED FIRST-ORDER APPROXIMATION LINE cx+b |
|---------|------------------------|--------------------------|-----------------------------------------------|
| 1 | 140 | 0.8 | -0.08×+100 |
| 2 | | | |
| ... | | | |

# FIG. 25

| MEAL ID | FEATURE AMOUNT 1 HEART RATE AT MEAL START TIME [bpm] | FEATURE AMOUNT 2 PEAK VALUE [bpm] | FEATURE AMOUNT 3 FIRST-ORDER APPROXIMATION LINE ax+b | FEATURE AMOUNT 4 DIGESTION FINISH TIME [hh:mm] | FEATURE AMOUNT 5 TIME BETWEEN MEAL FINISH TIME AND PEAK VALUE MEASUREMENT TIME [min] | FEATURE AMOUNT 6 TIME BETWEEN PEAK VALUE MEASUREMENT TIME AND DIGESTION FINISH TIME [min] |
|---|---|---|---|---|---|---|
| 1 | 80 | 100 | -0.1x+100 | 16:35 | | 250 |
| 2 | | | | | | |
| ... | | | | | | |

12C

EP 3 488 773 B1

# FIG. 26

EP 3 488 773 B1

12C

| MEAL ID | FEATURE AMOUNT 1 | FEATURE AMOUNT 2 | FEATURE AMOUNT 3 | FEATURE AMOUNT 4 | FEATURE AMOUNT 5 | FEATURE AMOUNT 6 |
|---------|---|---|---|---|---|---|
| | HEART RATE AT MEAL START TIME [bpm] | PEAK VALUE [bpm] | FIRST-ORDER APPROXIMATION LINE ax+b | DIGESTION FINISH TIME [hh:mm] | TIME BETWEEN MEAL FINISH TIME AND PEAK VALUE MEASUREMENT TIME [min] | TIME BETWEEN PEAK VALUE MEASUREMENT TIME AND DIGESTION FINISH TIME [min] |
| 1 | 80 | 100 | -0.1x+100 | 16:35 | 18 | 250 |
| 2 | | | | | | |
| ... | | | | | | |

# FIG. 27

S109

S110
IS IT TIME TO ESTIMATE
DIGESTIBILITY?

NO

YES

S111
CALCULATE DIGESTIBILITY AT
CURRENT TIME

S112
PRESENT INFORMATION TO USER?

NO

YES

S113
THERE IS CONTINUING SERVICE?

YES

NO

S114
PRESENT INFORMATION TO USER

END

# FIG. 28

12f

| CALCULATION TIME [hh:mm] | DIGESTIBILITY [%] |
|---|---|
| ... | |
| 12:30 | |
| 12:40 | |
| 12:50 | |
| 13:00 | |
| 13:10 | |
| 13:20 | |
| 13:30 | |
| 13:40 | |
| 13:50 | |
| 14:00 | |
| 14:10 | |
| 14:20 | |
| 14:30 | |
| ... | |

# FIG. 29

12f

| CALCULATION TIME [hh:mm] | DIGESTIBILITY [%] |
|---|---|
| ... | |
| 12:30 | 9 |
| 12:40 | |
| 12:50 | |
| 13:00 | |
| 13:10 | |
| 13:20 | |
| 13:30 | |
| 13:40 | |
| 13:50 | |
| 14:00 | |
| 14:10 | |
| 14:20 | |
| 14:30 | |
| ... | |

# FIG. 30

12f

| CALCULATION TIME [hh:mm] | DIGESTIBILITY [%] |
|---|---|
| . . . | |
| 12:30 | 9 |
| 12:40 | 12 |
| 12:50 | 16 |
| 13:00 | 20 |
| 13:10 | 24 |
| 13:20 | 27 |
| 13:30 | 31 |
| 13:40 | 35 |
| 13:50 | 38 |
| 14:00 | 42 |
| 14:10 | 46 |
| 14:20 | 50 |
| 14:30 | 53 |
| . . . | |

# FIG. 31

START

CALCULATE DIGESTIBILITY AT CURRENT TIME — S301

RECEIVE INFORMATION INDICATING THAT DIGESTIBILITY HAS REACHED 100% — S302

CALCULATE EATING ACTIVITY ENERGY — S303

CALCULATE DIFFERENCE BETWEEN DIGESTIBILITY CALCULATED IN S301 AND ACTUAL DIGESTIBILITY ACQUIRED IN S302 — S304

S305

DIFFERENCE IS 0? — YES

NO

CALCULATE CORRECTED VALUE OF SLOPE OF FIRST-ORDER APPROXIMATION LINE — S306

DETERMINE CORRESPONDING CORRECTED VALUE FOR EACH RANGE OF PREDETERMINED EATING ACTIVITY ENERGY — S307

END

# FIG. 32

(a)

| EATING ACTIVITY ENERGY | SLOPE | DIFFERENCE IN DIGESTIBILITY | CORRECTED VALUE $\alpha$ |
|---|---|---|---|
| ... | | | |
| 100 | -0.5 | -10 | |
| 120 | -0.5 | 0 | |
| 140 | -0.5 | -20 | |
| ... | | | |

(b)

| EATING ACTIVITY ENERGY | SLOPE | DIFFERENCE IN DIGESTIBILITY | CORRECTED VALUE $\alpha$ |
|---|---|---|---|
| ... | | | |
| 100 | -0.4 | 0 | 0.8 |
| 120 | -0.5 | 0 | 1.0 |
| 140 | -0.6 | 0 | 1.2 |
| ... | | | |

# FIG. 33

200

# FIG. 34

# FIG. 35

```
        ┌─────────────┐
        │    S106     │
        └─────────────┘
               │
               ▼
┌─────────────────────────────────────────┐
│  CALCULATE EATING ACTIVITY ENERGY        │──~S201a  ┐
└─────────────────────────────────────────┘          │
               │                                      │
               ▼                                      │
┌─────────────────────────────────────────┐          │
│  EXTRACT CORRECTED VALUE CORRESPONDING TO│          │
│  CALCULATED EATING ACTIVITY ENERGY BY    │──~S202a  ├─ S107
│  REFERRING TO CORRECTED VALUE MANAGEMENT │          │
│  TABLE                                   │          │
└─────────────────────────────────────────┘          │
               │                                      │
               ▼                                      │
┌─────────────────────────────────────────┐          │
│  CORRECT Y-INTERCEPT OF FIRST-ORDER      │──~S203a  │
│  APPROXIMATION LINE WITH EXTRACTED       │          │
│  CORRECTED VALUE                         │          ┘
└─────────────────────────────────────────┘
               │
               ▼
        ┌─────────────┐
        │   TO S108   │
        └─────────────┘
```

# FIG. 36

12h

| EATING ACTIVITY ENERGY | CORRECTED VALUE $\beta$ |
|---|---|
| ... | ... |
| 131 TO 150 | 20 |
| 111 TO 130 | 0 |
| 90 TO 110 | -20 |
| ... | ... |

# FIG. 37

12g

| MEAL ID | EATING ACTIVITY ENERGY | CORRECTED VALUE $\beta$ | CORRECTED FIRST-ORDER APPROXIMATION LINE ax+d |
|---------|------------------------|-------------------------|-----------------------------------------------|
| 1 | | | |
| 2 | | | |
| ... | | | |

# FIG. 38

12g

| MEAL ID | EATING ACTIVITY ENERGY | CORRECTED VALUE $\beta$ | CORRECTED FIRST-ORDER APPROXIMATION LINE ax+d |
|---|---|---|---|
| 1 | 140 | 20 | |
| 2 | | | |
| ... | | | |

# FIG. 39

12g

| MEAL ID | EATING ACTIVITY ENERGY | CORRECTED VALUE $\beta$ | CORRECTED FIRST-ORDER APPROXIMATION LINE ax+d |
|---------|------------------------|-------------------------|-----------------------------------------------|
| 1       | 140                    | 20                      | -0.1x+120                                      |
| 2       |                        |                         |                                               |
| ...     |                        |                         |                                               |

EP 3 488 773 B1

# FIG. 40

300

**HEART RATE SIGNAL MEASUREMENT DEVICE** ~20

10c

**INPUT UNIT** ~11

10d

**RECEPTION UNIT** ~13

**FIRST RECEIVER** 13a

**SECOND RECEIVER** 13b

**INFORMATION PROCESSOR**

14~ **FEATURE AMOUNT CALCULATION UNIT**

**DIGESTIBILITY ESTIMATION UNIT** ~15

16~ **DETERMINATION UNIT**

**CORRECTION UNIT** ~17

18~ **INFORMATION PRESENTATION UNIT**

**TABLE GENERATION UNIT** ~19

12

**HEART RATE MANAGEMENT TABLE** 12a

**MEAL TIME MANAGEMENT TABLE** 12b

**FEATURE AMOUNT MANAGEMENT TABLE** 12c

**FIRST-ORDER APPROXIMATION LINE MANAGEMENT TABLE** 12d

**CORRECTED VALUE MANAGEMENT TABLE** 12e

**DIGESTIBILITY ESTIMATION MANAGEMENT TABLE** 12f

**STORAGE UNIT**

**INFORMATION PROCESSOR**

# FIG. 4 1

```
                    ┌─────────┐
                    │  START  │
                    └─────────┘
                         │
                         ▼
┌─────────────────────────────────────────┐
│ RECEIVE PLURALITY OF PIECES OF HEART RATE│
│ INFORMATION FROM HEART RATE SIGNAL       │──  S401
│ MEASUREMENT DEVICE                       │
└─────────────────────────────────────────┘
                         │
                         ▼
┌─────────────────────────────────────────┐
│ ACQUIRE MEAL START TIME AND              │──  S402
│ MEAL FINISH TIME INFORMATION             │
└─────────────────────────────────────────┘
                         │
                         ▼
┌─────────────────────────────────────────┐
│ SPECIFY HEART RATE AT MEAL START TIME    │──  S403
└─────────────────────────────────────────┘
                         │
                         ▼
┌─────────────────────────────────────────┐
│ SPECIFY HEART RATE PEAK VALUE AFTER MEAL │──  S404
└─────────────────────────────────────────┘
                         │
         ┌───────────────┤
         │               ▼
         │          ╱─────────────────────╲      S405
         │  NO     ╱  FIRST-ORDER           ╲
         └────────(   APPROXIMATION          )
                   ╲  LINE MAY BE CALCULATED? ╱
                    ╲─────────────────────────╱
                         │ YES
                         ▼
┌─────────────────────────────────────────┐
│ CALCULATE FIRST-ORDER APPROXIMATION LINE │──  S406
└─────────────────────────────────────────┘
                         │
                         ▼
┌─────────────────────────────────────────┐
│ CORRECT FIRST-ORDER APPROXIMATION LINE   │──  S407
└─────────────────────────────────────────┘
                         │
                         ▼
┌─────────────────────────────────────────┐
│ SPECIFY DIGESTION FINISH TIME            │──  S408
└─────────────────────────────────────────┘
                         │
                         ▼
┌─────────────────────────────────────────┐
│ CALCULATE TIME BETWEEN MEAL FINISH TIME  │──  S409
│ AND PEAK VALUE MEASUREMENT TIME          │
└─────────────────────────────────────────┘
                         │
                         ▼
                    ┌─────────┐
                    │ TO S110 │
                    └─────────┘
```

# FIG. 42

S409

S410
IS IT TIME TO ESTIMATE DIGESTIBILITY?
NO
YES

S411
CALCULATE DIGESTIBILITY AT CURRENT TIME

S412
PRESENT INFORMATION TO USER?
NO
YES

S413
THERE IS CONTINUING SERVICE?
YES
NO

S414
PRESENT INFORMATION TO USER

END

# FIG. 43

400

# FIG. 44

```
                    ┌─────────────┐
                    │    START    │
                    └─────────────┘
                           │
                           ▼               ⌇S501
                    ╱───────────────╲
              YES  ╱  EATING ACTIVITY  ╲  NO
         ┌───────╱ ENERGY<THRESHOLD?     ╲───────┐
         │       ╲                       ╱        │
         │        ╲─────────────────────╱         │
         ▼                                        ▼
  ⌇S502                                    ⌇S503
┌─────────────────────────┐        ┌─────────────────────────┐
│ PREFERENTIALLY DISPLAY  │        │ PREFERENTIALLY DISPLAY  │
│  EATING PLACE ALLOWING  │        │  EATING PLACE ALLOWING  │
│  SLOW EATING ON SCREEN  │        │ SPEED-EATING ON SCREEN  │
└─────────────────────────┘        └─────────────────────────┘
         │                                        │
         ▼◄───────────────────────────────────────┘
  ┌─────────┐
  │   END   │
  └─────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10504739 A
- JP 2004000138 A
- JP 2009201805 A
- JP 2011115508 A
- JP 2008061790 A
- EP 3354193 A1
- WO 2016092707 A1

**Non-patent literature cited in the description**

- Diet Support Service by DOCOMO - Display Calories from Meal Photo and Measure Hungry Level from Breath. ITmedia Inc, 06 July 2015